# EUROPEAN PATENT APPLICATION

(11) **EP 4 725 943 A1**
(43) Date of publication of application: **15.04.2026**
(21) Application number: 24206538.1
(22) Date of filing: 14.10.2024
(51) Int. Cl.: C07D 215/14, C07D 217/26, C07D 215/54, C07D 333/38, A61P 35/00, A61K 31/472

(54) **INHIBITORS OF LACTATE DEHYDROGENASE**

(71) Applicant: PharmAI Discovery GmbH, 01067 Dresden (DE); Université catholique de Louvain, 1348 Louvain-la-Neuve (BE)
(72) Inventor: SONVEAUX, Pierre, 1933 Sterrebeek (BE); FRÉDÉRICK, Raphaël, 5530 Godinne (BE); SPILLIER, Quentin, 5300 Coutisse (BE); KLUGE, Andreas, 01326 Dresden (DE); KAISER, Florian Tobias, 08258 Markneukirchen (DE); LEBERECHT, Christoph, 01936 Schwepnitz (DE); HAUPT, Joachim, 09627 Bobritzsch-Hilbersdorf (DE)
(74) Representative: Vossius & Partner Patentanwälte Rechtsanwälte mbB

(57) **Abstract**

The present invention relates to a compound of formula (1), (2) or (3), wherein formula (1) is 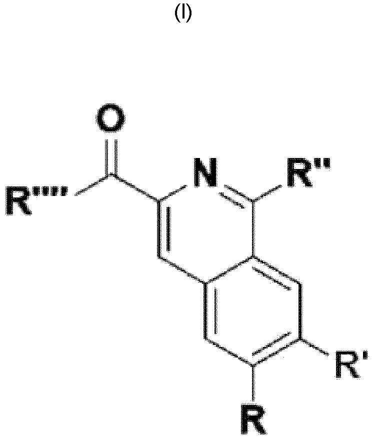 (formula (1)), wherein
R is -H, -F or -Cl;
R' is -H, -F or -Cl;
R" is 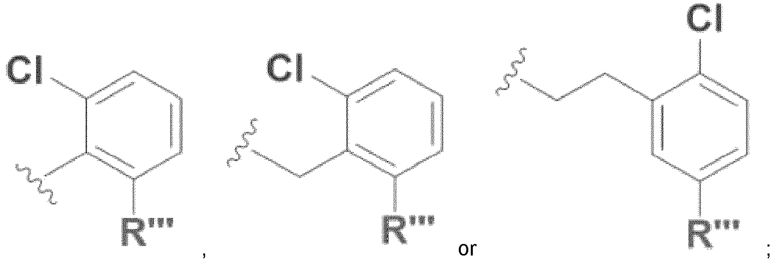 R‴ is -H or -OH; and
Rʺʺ is 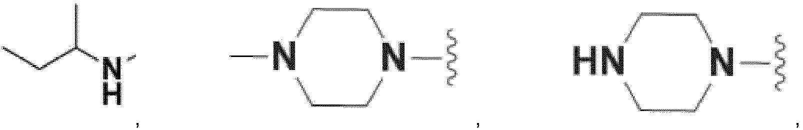 n= 1,2,3 n= 1,2,3 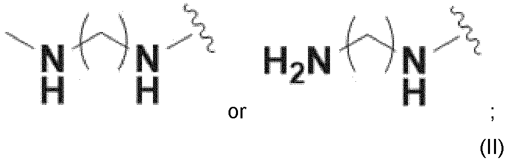 formula (2) is 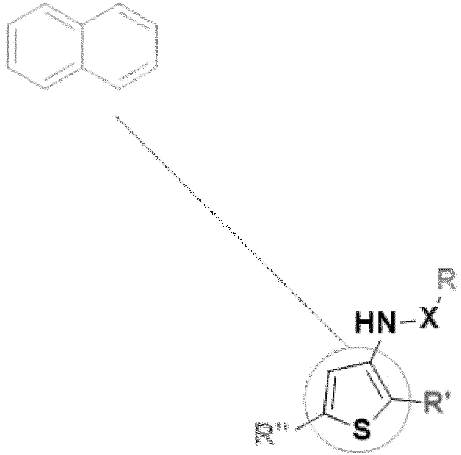 (formula (2)), wherein;
R is 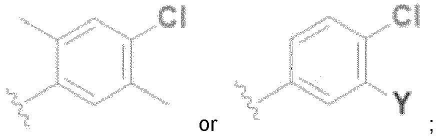 X is 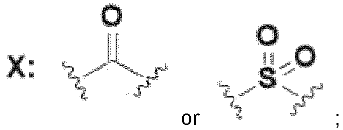 Y is -OH, -OCH₃ or CH₃;
R' is 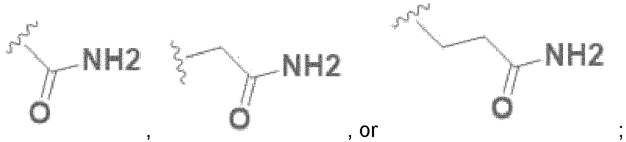 and
R" is 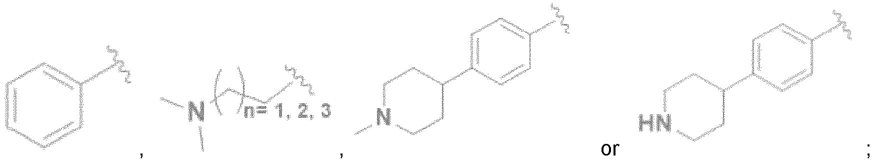 and
formula (III) is

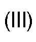

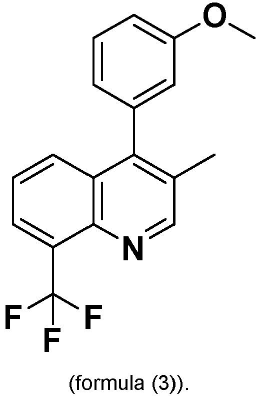

## Description

The present invention relates to a compound of formula (1), (2) or (3), wherein formula (1) is 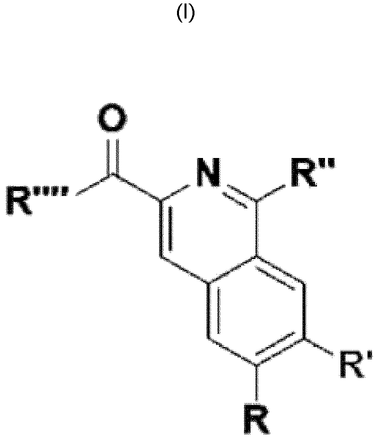 (formula (1)), wherein
R is -H, -F or -Cl;
R' is -H, -F or -Cl;
R" is 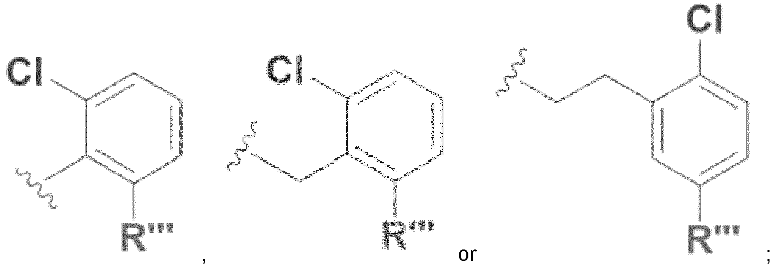
R‴ is -H or -OH; and
R"" is 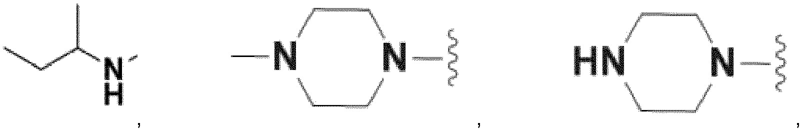 n= 1,2,3 n= 1,2,3 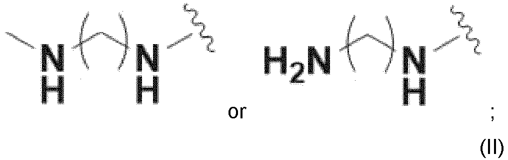
formula (2) is 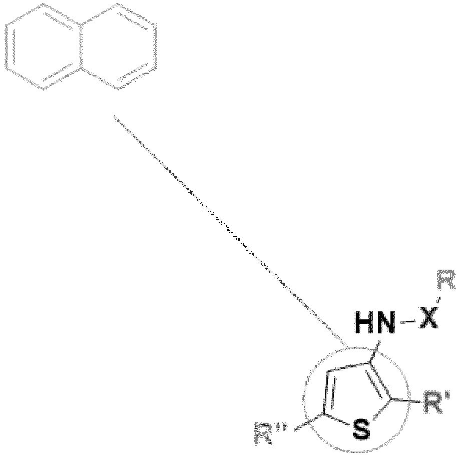 (formula (2)), wherein;
R is 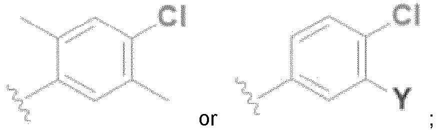
X is 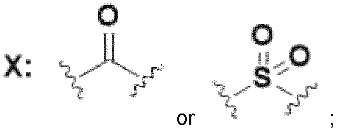
Y is -OH, -OCH₃ or CH₃;
R' is 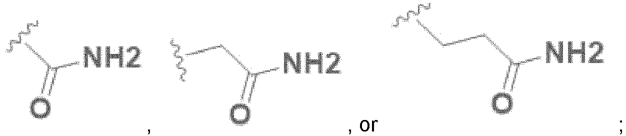 and
R" is 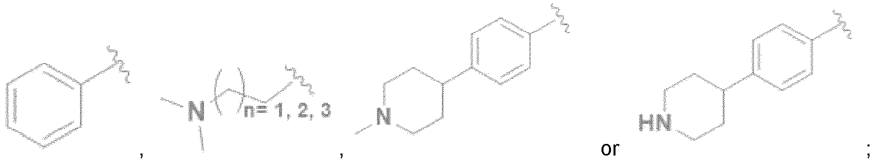 and
formula (III) is

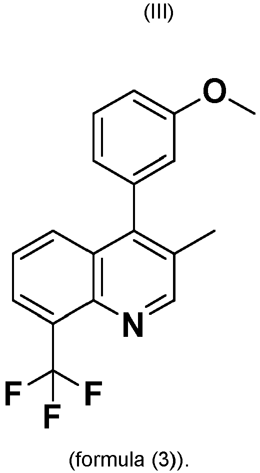

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

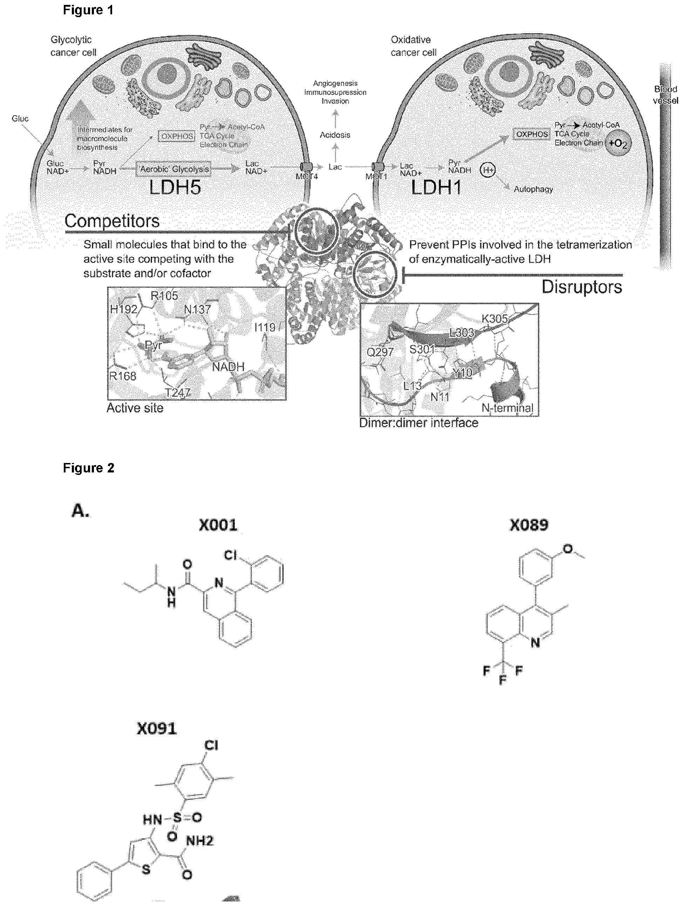

Lactate dehydrogenase (LDH) catalyzes the reversible conversion of lactate + NAD⁺ to pyruvate + NADH + H⁺. In tumors, LDH plays a central role in the metabolic symbiosis based on lactate exchange, thereby contributing to cancer progression (Figure 1). Moreover, the identification that lactate stimulates angiogenesis in wounds and in tumors, and evidences that lactate dehydrogenase B (LDHB) is essential for autophagy in cancer cells have triggered the discovery of potent and selective LDH inhibitors. This possibility is grounded on collaborative works with the team of Pierre Sonveaux, showing that LDHB silencing with shRNAs kills many types of human cancer cell lines (i.e. breast, colon and cervix cancers and glioblastomas) while sparing normal adult human cells.¹

So far however, all the small molecules developed against LDH revealed to be poorly active and/or deprived of the required selectivity profile. Indeed, LDH is known to be very difficult and poorly druggable not only because of its highly hydrophilic and size-limited active site cavity but also because of its high cellular concentrations.^{5, 6} Since LDH is active as tetramer, our team pioneered on a completely different approach for LDH inhibition which aims at the design of molecules able to disrupt and/or prevent this tetramerization process (Figure 1).

Besides our works, recent studies reported novel allosteric strategies to inhibit LDH, using a wide range of molecules and mechanisms of action. These strategies include, but are not limited to, targeting PPIs between LDH subunits involved in the oligomerization of LDH. Therefore, multiple regions leading to allosteric inhibition of LDH have hence been identified.

LDH tetramerizes by dimerization of dimers, and the N-terminal arm of LDH was identified as a key region in this dimer:dimer interface. Jafary et al. designed a set of peptides *in silico* to target this interface.⁷ The 6-mer peptide QLIYNL showed a reduction in the average size of LDH, suggesting a loss of the tetrameric form.⁷ Our team reported LT26, a stapled α-helical peptide that mimics the α-helix of the N-terminal arm and binds to this interface, destabilizing LDH.² Moreover, a biophysical screening of FDA-approved drugs identified Maprotiline and Triprolidine, two small molecules able to bind at slightly different spots of this dimer:dimer interface and impact the stability of the LDH tetrameric structure.⁴ Soon after we reported our discovery of LT26, Nadal-Bufi et al., reported cGmC9, a β-hairpin peptide that targets the β-sheet located in this interface. Strikingly this cGmC9 compound showed potent inhibition of LDH enzymatic activity.⁸ A follow-up work from the same team, reported dcGmC9 which showed enhanced cell-penetrating properties (Table 1).⁹

| **Table 1: Reported allosteric inhibitors of lactate dehydrogenase** | | | | | |
|---|---|---|---|---|---|
| **Inhibitor** | **Class** | **Reported binding region** | **Crystal.** | **Disruption** | **Ref.** |
| QLIYNL | Peptide | N-terminal arm, Dimer:dimer interface 1 | No | NA | 7 |
| LT26 | Stapled α-helical peptide | N-terminal arm, Dimer:dimer interface 1 | No | Yes | 2 |
| Maprotiline | Small molecule | N-terminal arm, Dimer:dimer interface 1 | Yes | NA | 4 |
| Triprolidine | Small molecule | N-terminal arm, Dimer:dimer interface 1 | No | NA | 4 |
| cGmC9 | B-hairpin peptide | B-sheet of the N-terminal arm, Dimer:dimer interface 1 | No | Yes | 8 |
| dcGmC9 | B-hairpin peptide | B-sheet of the N-terminal arm, Dimer:dimer interface 1 | No | Yes | 9 |
| GP16 | Stapled α-helical peptide | Dimer:dimer interface 2 | No | Yes | 3 |
| phthalimide | Small molecule | Dimer:dimer interface 2 | Yes | No | 10 |
| dibenzofuran | Small molecule | Dimer:dimer interface 2 | Yes | No | 10 |
| AXKO-0046 | Small molecule | Monomer:monomer interface | Yes | No | 11 |
| FLCN-10 | Peptide | Active-site loop | No | No | 12 |

In parallel to these works targeting the N-terminal arm of LDH involved in the LDH tetramerization process, other allosteric strategies to inhibit LDH were reported. Interestingly, we identified a second dimer:dimer interface that would contribute to the LDH tetramer formation.³ This was validated by the production and evaluation of LDH bearing mutations on this region (residues 61-76) that failed to display a prototypical tetrameric structure. Furthermore, GP16, a stapled peptide designed to bind to this interface, impacted the stability of the enzyme.³

In addition, Friberg et al. recently reported small molecules phthalimide and dibenzofuran as allosteric inhibitors of LDH.¹⁰ Without providing explicit studies on the stability of the tetramer, co-crystal structures of this compounds with LDH revealed that their binding site is exactly at the second identified dimer:dimer interface, same as GP16 (Table 1).

Shibata et al. reported the small molecule AXKO-0046 which showed uncompetitive inhibition of LDH. Interestingly, its co-crystal structure reveals that AXKO-0046 binds exactly at the monomer:monomer interface of LDH, uncovering a third allosteric interface.¹¹ In fact, the authors of the study speculate that its mechanism of action may be related with the destabilization of the protein (Table 1).

Finally, FLCN-10, a peptide isolated from the tumor suppressor folliculin, also showed allosteric inhibition of LDH. However, its binding site is not on a reported oligomeric interface.¹² A similar allosteric strategy was reported by Dzierlenga et al., and Andrews et al., which reported small molecules that inhibit enzymatic activity by interacting with a key flexible loop of the active site, without binding directly to the active site (Table 1).^{13, 14}

Hence, although some LDH inhibitors are available in the prior art there is still an ongoing need for novel LDH inhibitors and in particular novel small molecules LDH inhibitors that are active against LDH in its tetrameric form. This need is addressed herein.

Accordingly the present invention relates in a first aspect to a compound of formula (1), (2) or (3), wherein
(I)
formula (1) is 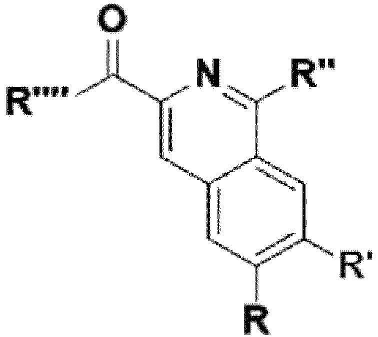 (formula (1)), wherein
R is -H, -F or -Cl;
R' is -H, -F or -Cl;
R" is 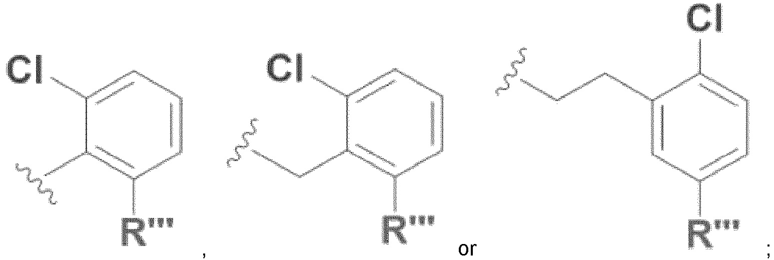
R‴ is -H or -OH; and
R"" is 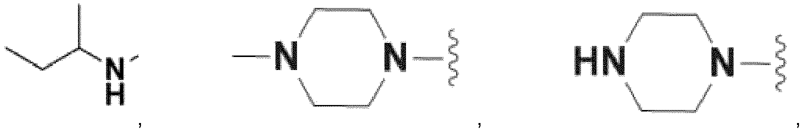 n= 1,2,3 **n=** 1,2,3 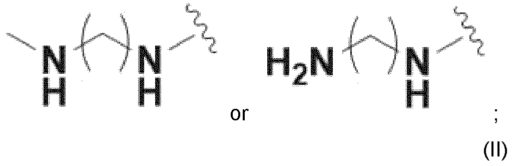
formula (2) is 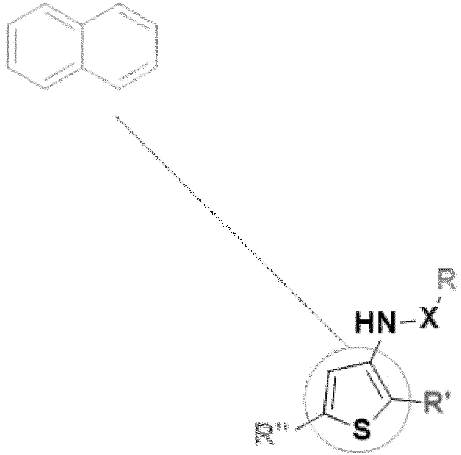 (formula (2)), wherein;
R is 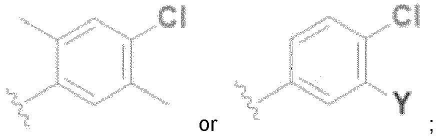
X is 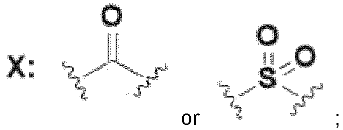
Y is -OH, -OCH₃ or CH₃;
R' is 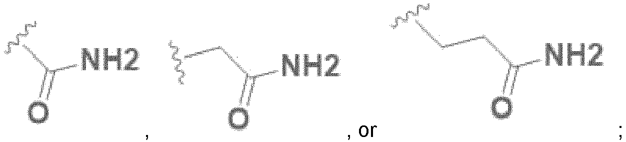 and
R" is 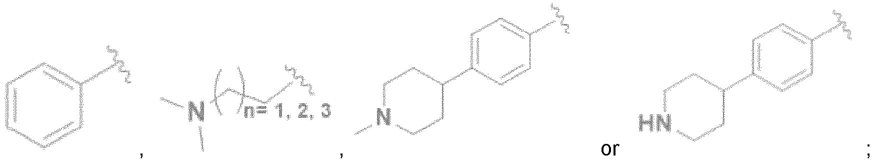 and
formula (III) is

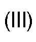

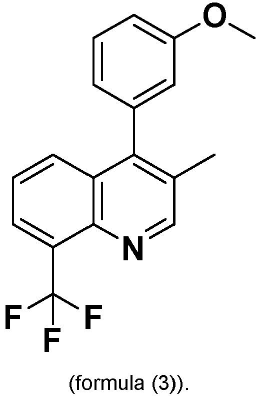

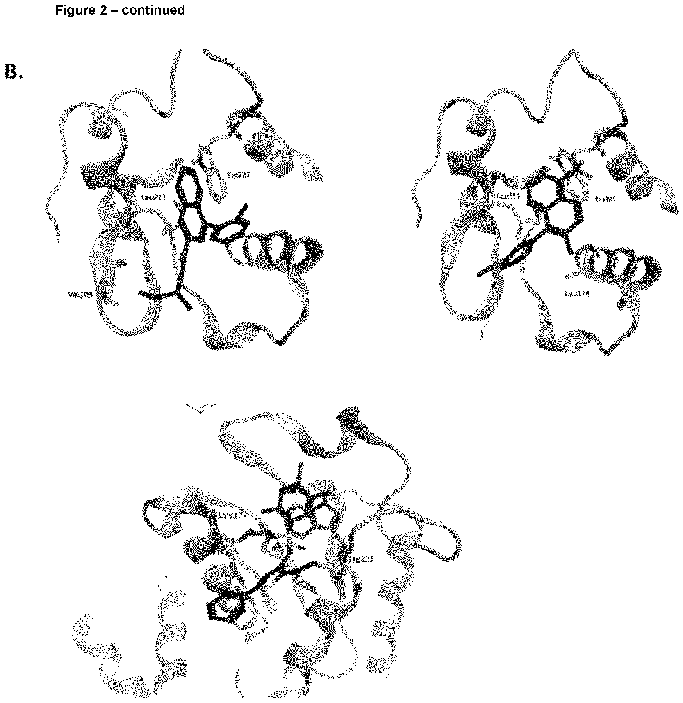

It is shown in the appended examples that the three molecules X001, X089 and X091 exhibit remarkable binding affinity to the LDH tetramerization site by *in silico* modelling (Figure 2B). X001 and X091 demonstrated inhibitory activity against three enzymatic forms of LDH (LDHA full-length subunit, LDHB full-length subunit and LDHB tetramer (LDHBtr)) in enzyme assays, with IC₅₀ values in the low micromolar range. X089 also demonstrated its ability to inhibit LDHA and LDHB full length in the micromolar range but without acting on LDHBtr, thereby indicating a different mode of action. Notably, X001 and X091 demonstrated LDHA inhibition following the dissociation-reassociation process within the same range observed in a conventional enzymatic assay. However, when the enzyme was not dissociated, the inhibition was completely abolished. In contrast, a tested reference compound from Genentech maintained consistent inhibition in this assay, regardless of protein dissociation. For X089, no inhibition (before or after dissociation) could be observed using this assay, again suggesting a different mode of action from the other two molecules X001 and X091 and from the reference orthosteric inhibitor. These findings suggest that X001 and X091 affect thermal stability LDHs and that they inhibit their activity by interacting at the tetrameric interface. In addition, in the examples the capacity of X001, X089 and X091 to selectively impede the proliferation of cancerous cell lines without exerting excessive impact on the healthy cell line is demonstrated. This shows the potential of the three compounds as candidates for designing novel anti-cancer therapies targeting LDH inhibition and for X001 and X091 in particular through the disruption of the tetrameric form of LDH.

Based on compound X001 and X091 and the identified binding interface between compounds X001 and X091 and LDH structurally related compounds were identified *in silico* that are likewise capable of inhibiting LDH, in particular the tetrameric form of LDH (Example 3). Formula (1) is the core structure of the "X001 compounds" and variable positions list the variable groups that characterize X001 and that were identified in the *in silico* screen. Similarly, formula (2) is the core structure of the "X091 compounds" and variable positions list the variable groups that characterize X091 and that were identified in the *in silico* screen.

With respect to formula (2) it is noted that the core structure of a thiophene ring can be replaced by a naphthalene ring, while the thiophene ring is preferred. This is indicated in formula (2) by the circle and line pointing away from the Markush core formula with its thiophene ring to the naphthalene ring. On the naphthalene ring the substituents -HN-X-R, -R' and -R" can preferably be found at carbon positions 1,2,5- or 1,2-6 of the naphthalene ring and hence, starting with position 1 as the -HN-X-R function, position 2 as the -R' and position 5 or 6 as the -R" function.

Based on the factors such as energy optimization (e.g., docking scores) and structural similarities to the originally tested compounds X001 and X091 it can be safely assumed that all compounds falling under formula (1) and (2) of the above first aspect are LDH inhibitors, in particular LDH inhibitors that can disrupt the tetrameric form of LDH.

Moreover formula (3) is compound X089, since the *in silico* modelling based on X089 did not reveal further LDH inhibitors. As discussed in the appended examples the exact mode of LDH inhibition by compound X089 is not yet fully understood. For this reason with the first aspect the compounds of formulas (1) and (2) are preferred as compared to the compound of formula (3). Similarly, compounds X001 and X091 are preferred as compared to compound X089.

While the prior art to the best knowledge of the inventors failed to provide small molecules against LDH that display a satisfactory active and selectivity profile, the compounds falling under the above first aspect overcome this prior art deficiency.

In accordance with a preferred embodiment of a first aspect to the compound of formula (1) one or more of (i) to (v) applies:
(i) R is -H;
(ii) R' is -H;
(iii) R" is 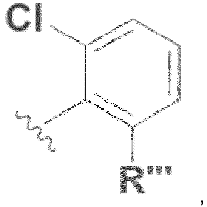 or;
(iv) R‴ is -H; and
(v) R"" is 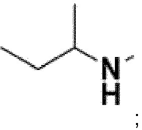 or
to the compound of formula (2) one or more of (i) to (vi) applies:
(i) R is 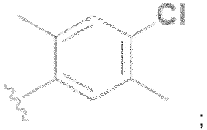
(ii) X is 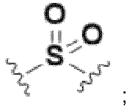
(iii) R' is 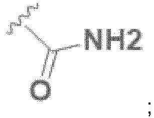 and
(iv) R" is

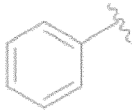

Items (i) to (v) list for each of the variable positions of formula (1) the residues that can be found in compound X001 at these variable positions. Items (i) to (v) are with increasing preference two or more, three of more, four or more, and all five items.

Items (i) to (vi) list for each of the variable positions of formula (2) the residues that can be found in compound X091 at these variable positions. Items (i) to (vi) are with increasing preference two or more, three of more, four or more, five or more and all six items.

In accordance with a further preferred embodiment of a first aspect the compound of formula (1) is 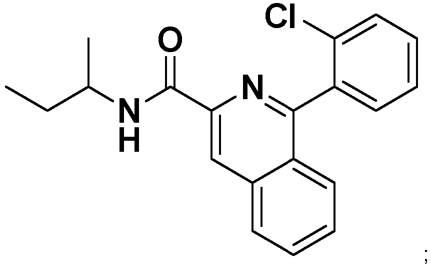 or the compound of formula (2) is

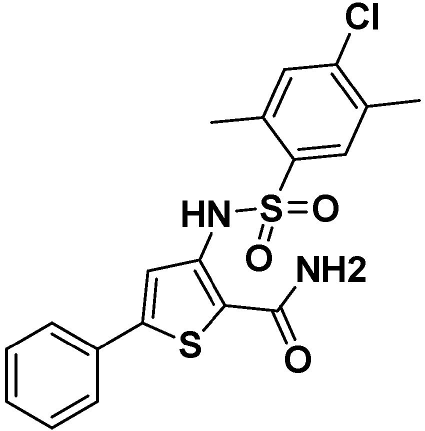

The two compounds according to this preferred embodiment are compounds X001 and X091, respectively.

In accordance with a preferred embodiment of a first aspect
(I) the compound of formula (1) or (2) has a chemical structure being selected from the list of compounds (1) to (191), preferably compounds (1) to (100), more preferably compounds (1) to (50), even more preferably compounds (1) to (25) and most preferably compounds (1) to (10) in Table 5 (IUPAC names) below.

Similarly, in accordance with a preferred embodiment of a first aspect
(I) the compound of formula (1) or (2) has a SMILES (Simplified Molecular Input Line Entry System) chemical structure being selected from the list of compounds (1) to (191), preferably compounds (1) to (100), more preferably compounds (1) to (50), even more preferably compounds (1) to (25) and most preferably compounds (1) to (10) in Table 6 below.

Tables 5 is shown in Example 3 herein below. Table 5 lists the IUPAC names of the 191 concrete LDH inhibitors that were identified in the *in silico* screen based on compounds X001 and X089, and the identified binding interface between compound X001 and LDH or X091 and LDH. Similarly, Table 6 lists the SMILES (Simplified Molecular Input Line Entry System) chemical structure of these 191 concrete LDH inhibitors.

Tables 5 and 6 list the compounds in the order of their predicted activity as LDH inhibitors. For this reason compounds (1) to (100) are preferred, compounds (1) to (50) are more preferred, compounds (1) to (25) are even more preferred and compounds (1) to (10) as shown in Table 5 and 6 below are most preferred, respectively.

Table 7 provides for each of the 191 concrete LDH inhibitors the information whether it is derived from compound X001 or X089. 96 concrete LDH inhibitors are derived from compound X001 while 95 compounds are derived from compound X089. Within the 96 concrete LDH inhibitors that are derived from compound X001 the first listed compounds (1) to (50) are preferred, compounds (1) to (25) are more preferred compounds (1) to (10) are even more preferred, and compounds (1) to (5) are most preferred. Similarly, within the 95 concrete LDH inhibitors that are derived from compound X091 the first listed compounds (1) to (50) are preferred, compounds (1) to (25) are more preferred compounds (1) to (10) are even more preferred, and compounds (1) to (5) are most preferred.

Table 7 also provides for each of the 191 concrete LDH inhibitors the information whether it clusters with other concrete LDH inhibitors. The most preferred clusters of LDH inhibitors that are derived from compound X001 are clusters 2 and/or 0, and most preferred clusters of LDH inhibitors that are derived from compound X091 are clusters 13 and/or 11.

In accordance with a preferred embodiment of the first aspect the compound is an inhibitor of LDH (lactate dehydrogenase, E.C. 1.1.1.27).

As discussed above, the compound of the first aspect is an inhibitor of LDH, in particular of the tetrameric form of LDH. Lactate dehydrogenase (LDH) is an enzyme found in nearly all living cells. LDH catalyzes the conversion of pyruvate to lactate and back, as it converts NAD⁺ to NADH and back. A dehydrogenase is an enzyme that transfers a hydrogen from one molecule to another.

LDH in humans uses His(193) as the proton acceptor, and works in unison with the coenzyme (Arg99 and Asn138), and substrate (Arg106; Arg169; Thr248) binding residues. The His(193) active site is not only found in the human form of LDH, but is found in many different animals, showing the convergent evolution of LDH. The two different subunits of LDH (LDHA, also known as the M subunit of LDH, and LDHB, also known as the H subunit of LDH) both retain the same active site and the same amino acids participating in the reaction. The noticeable difference between the two subunits that make up LDH's tertiary structure is the replacement of alanine (in the M chain) with a glutamine (in the H chain). This tiny but notable change is believed to be the reason the H subunit can bind NAD faster, and the M subunit's catalytic activity is not reduced in the presence of acetylpyridine adenine dinucleotide, whereas the H subunit's activity is reduced fivefold.

Enzymatically active lactate dehydrogenase consists of four subunits (tetramer). The two most common subunits are the LDH-M (or LDHA) and LDH-H (or LDHB) peptides, named for their discovery in muscle and heart tissue, and encoded by the LDHA and LDHB genes, respectively. These two subunits can form five possible tetramers (isoenzymes): LDH-1 (4H), LDH-5 (4M), and the three mixed tetramers (LDH-2/3H1M, LDH-3/2H2M, LDH-4/1H3M). These five isoforms act enzymatically similarly but show different tissue distribution.
LDH-1 (4H): in the heart and in RBC (red blood cells), as well as the brain
LDH-2 (3H1M): in the reticuloendothelial system
LDH-3 (2H2M): in the lungs
LDH-4 (1H3M): in the kidneys, placenta, and pancreas
LDH-5 (4M): in the liver and striated muscle, also present in the brain

The LDH is preferably human LDH. The mRNA and protein sequences of human LDHA can found at NCBI Reference Sequence NM_005566.4 while the mRNA and protein sequences of human LDHB can found at NCBI Reference Sequence NM_002300.8.

In accordance with a more preferred embodiment of the first aspect the inhibitor inhibits the formation of the enzymatically active tetrameric form of LDH.

As discussed herein above, X001 and X089 are shown in the appended examples to inhibit the formation of the enzymatically active tetrameric form of LDH. Hence, the inhibitor of this more preferred embodiment is a compound of formula (1) or formula (2) as defined herein above and is preferably X001 and X089.

The LDH tetrameric interface is known from Thabault et al. (2021), J Biol Chem., 296: 100422. Here, E62, D65, L71, and F72 were found to be essential for LDH tetrameric stability and the crystal structure of active LDH can be found in the Protein Data Bank ID 1I0Z. E62 and D65 are negatively charged amino acids, and L71, F72, and L73 are three consecutive hydrophobic residues.

In accordance with another more preferred embodiment of the first aspect the LDH inhibitor displays an IC₅₀ for LDH of 100 µM of less, preferably 50µM or less for human LDHA and/or LDHB.

The compounds X001, X089 and X091 even displays an IC₅₀ value in the low micromolar range for human LDHA and/or LDHB (Example 2).

In accordance with a more preferred embodiment of the first aspect the LDH (i) comprises or consists of the amino acid sequence of SEQ ID NO: 1 or 2, or a sequence being at least 90% (such as at least 95%) identical thereto; or (ii) comprises or consists of an amino acid sequence being encoded by the nucleotide sequence of SEQ ID NO: 3 or 4, or a sequence being at least 90% (such as at least 95%) identical thereto.

The amino acid sequence of SEQ ID NO: 1 or 2 are the amino acid sequence of the human LDHA and LDHB proteins, while the nucleotide sequence of SEQ ID NO: 3 or 4 are nucleotide sequence of the human LDHA and LDHB genes.

In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 90% or 95% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Nucleotide and amino acid sequence analysis and alignment in connection with the present invention are preferably carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), Nucleic Acids Res. 25:3389-3402). BLAST can be used for nucleotide sequences (nucleotide BLAST) and amino acid sequences (protein BLAST). The skilled person is aware of additional suitable programs to align nucleic acid sequences.

As defined herein, sequence identities of at least 90% are envisaged by the invention. However, also envisaged by the invention are with increasing preference sequence identities of at least 95%, at least 97.5%, at least 98.5%, at least 99%, at least 99.5%, at least 99.8%, and 100%.

The present invention relates in a second aspect to a conjugate comprising the compound of the first aspect conjugated to a peptide or protein, preferably a pharmaceutically active peptide or protein.

The term "protein" as used herein interchangeably with the term "polypeptide" describes linear molecular chains of amino acids, including single chain proteins or their fragments, containing at least 50 amino acids. The term "peptide" as used herein describes a group of molecules consisting of up to 49 amino acids, whereas the term "polypeptide" (also referred to as "protein") as used herein describes a group of molecules consisting of at least 50 amino acids. The term "peptide" as used herein describes a group of molecules consisting with increased preference of at least 15 amino acids, at least 20 amino acids at least 25 amino acids, and at least 40 amino acids. The group of peptides and polypeptides are referred to together by using the term "(poly)peptide". (Poly)peptides may further form oligomers consisting of at least two identical or different molecules. The corresponding higher order structures of such multimers are, correspondingly, termed homo- or heterodimers, homo- or heterotrimers etc.. Furthermore, peptidomimetics of such proteins/(poly)peptides where amino acid(s) and/or peptide bond(s) have been replaced by functional analogues are also encompassed by the invention. Such functional analogues include all known amino acids other than the 20 gene-encoded amino acids, such as selenocysteine. The terms "(poly)peptide" and "protein" also refer to naturally modified (poly)peptides and proteins where the modification is effected e.g. by glycosylation, acetylation, phosphorylation and similar modifications which are well known in the art.

A pharmaceutically active peptide or protein designates a peptide or protein that when being administered to a subject can exert a disease preventive and/or disease curative effect. Also envisioned herein is a diagnostically active peptide or protein. A diagnostically active peptide or protein when being administered to a subject can indicate the presence and/or location of disease.

Means and method for conjugating peptide or protein, preferably a pharmaceutically active peptide or protein to small molecules, such the compounds of the first aspect are known in the art. For example, click chemistry can be used. Click chemistry reaction reagents enable researchers to covalently link virtually any unnatural chemical groups, including inorganic catalysts or photosensitizers, to any site of a target protein with high site-selectivity; see, for example, C. Hu, J. Wang, in Methods in Enzymology, Volume 580, 2016, Pages 109-133.

In accordance with a preferred embodiment of the second aspect the peptide or protein is an antibody or an antibody mimetic, preferably an anti-tumor antigen antibody or antibody mimetic.

The term "antibody" as used in accordance with the present invention comprises, for example, polyclonal or monoclonal antibodies. Furthermore, also derivatives or fragments thereof, which still retain the binding specificity to the target, e.g. a tumor antigen, are comprised in the term "antibody". Antibody fragments or derivatives comprise, *inter alia,* Fab or Fab' fragments, Fd, F(ab')2, Fv or scFv fragments, single domain VH or V-like domains, such as VhH or V-NAR-domains, as well as multimeric formats such as minibodies, diabodies, tribodies or triplebodies, tetrabodies or chemically conjugated Fab'-multimers (see, for example, Harlow and Lane "Antibodies, A Laboratory Manual", Cold Spring Harbor Laboratory Press, 198; Harlow and Lane "Using Antibodies: A Laboratory Manual" Cold Spring Harbor Laboratory Press, 1999; Altshuler EP, Serebryanaya DV, Katrukha AG. 2010, Biochemistry (Mosc)., vol. 75(13), 1584; Holliger P, Hudson PJ. 2005, Nat Biotechnol., vol. 23(9), 1126). The multimeric formats in particular comprise bispecific antibodies that can simultaneously bind to two different types of antigen. The first antigen can be found on the protein of the invention. The second antigen may, for example, be a tumor marker that is specifically expressed on cancer cells or a certain type of cancer cells. Non-limting examples of bispecific antibodies formats are Biclonics (bispecific, full length human IgG antibodies), DART (Dual-affinity Re-targeting Antibody) and BiTE (consisting of two single-chain variable fragments (scFvs) of different antibodies) molecules (Kontermann and Brinkmann (2015), Drug Discovery Today, 20(7):838-847).

The term "antibody" also includes embodiments such as chimeric (human constant domain, non-human variable domain), single chain and humanised (human antibody with the exception of non-human CDRs) antibodies.

Various techniques for the production of antibodies are well known in the art and described, e.g. in Harlow and Lane (1988) and (1999) and Altshuler et al., 2010, loc. cit. Thus, polyclonal antibodies can be obtained from the blood of an animal following immunisation with an antigen in mixture with additives and adjuvants and monoclonal antibodies can be produced by any technique which provides antibodies produced by continuous cell line cultures. Examples for such techniques are described, e.g. in Harlow E and Lane D, Cold Spring Harbor Laboratory Press, 1988; Harlow E and Lane D, Using Antibodies: A Laboratory Manual, Cold Spring Harbor Laboratory Press, 1999 and include the hybridoma technique originally described by Köhler and Milstein, 1975, the trioma technique, the human B-cell hybridoma technique (see e.g. Kozbor D, 1983, Immunology Today, vol.4, 7; Li J, et al. 2006, PNAS, vol. 103(10), 3557) and the EBV-hybridoma technique to produce human monoclonal antibodies (Cole et al., 1985, Alan R. Liss, Inc, 77-96). Furthermore, recombinant antibodies may be obtained from monoclonal antibodies or can be prepared de novo using various display methods such as phage, ribosomal, mRNA, or cell display. A suitable system for the expression of the recombinant (humanised) antibodies may be selected from, for example, bacteria, yeast, insects, mammalian cell lines or transgenic animals or plants (see, e.g., US patent 6,080,560; Holliger P, Hudson PJ. 2005, Nat Biotechnol., vol. 23(9), 11265). Further, techniques described for the production of single chain antibodies (see, inter alia, US Patent 4,946,778) can be adapted to produce single chain antibodies specific for a epitope. Surface plasmon resonance as employed in the BIAcore system can be used to increase the efficiency of phage antibodies.

As used herein, the term "antibody mimetics" refers to compounds which, like antibodies, can specifically bind antigens, such a tumor antigen, but which are not structurally related to antibodies. Antibody mimetics are usually artificial peptides or proteins with a molar mass of about 3 to 20 kDa. For example, an antibody mimetic may be selected from the group consisting of affibodies, adnectins, anticalins, DARPins, avimers, nanofitins, affilins, Kunitz domain peptides, Fynomers^{®}, trispecific binding molecules and prododies. These polypeptides are well known in the art and are described in further detail herein below.

Conjugates with an antibody or antibody mimetic can be produced by the same chemical reactions that are used to prepare antibody drug conjugates. Antibody drug conjugates (ADCs) are synthesized by conjugating a drug or "payload" to a monoclonal antibody. The payloads can, for example, be conjugated using amino or sulfhydryl specific linkers that react with lysines or cysteines on the antibody surface; see Jackson, 2016, Organic Process Research & Development, Vol 20/Issue 5.

In accordance with a more preferred embodiment of the second aspect the antibody mimetic is selected from affibodies, adnectins, anticalins, DARPins, avimers, nanofitins, affilins, Kunitz domain peptides, Fynomers, trispecific binding molecules and probodies.

The term "affibody", as used herein, refers to a family of antibody mimetics which is derived from the Z-domain of staphylococcal protein A. Structurally, affibody molecules are based on a three-helix bundle domain which can also be incorporated into fusion proteins. In itself, an affibody has a molecular mass of around 6kDa and is stable at high temperatures and under acidic or alkaline conditions. Target specificity is obtained by randomisation of 13 amino acids located in two alpha-helices involved in the binding activity of the parent protein domain (Feldwisch J, Tolmachev V.; (2012) Methods Mol Biol. 899:103-26).

The term "adnectin" (also referred to as "monobody"), as used herein, relates to a molecule based on the 10th extracellular domain of human fibronectin III (10Fn3), which adopts an Ig-like β-sandwich fold of 94 residues with 2 to 3 exposed loops, but lacks the central disulphide bridge (Gebauer and Skerra (2009) Curr Opinion in Chemical Biology 13:245-255). Adnectins with the desired target specificity, can be genetically engineered by introducing modifications in specific loops of the protein.

The term "anticalin", as used herein, refers to an engineered protein derived from a lipocalin (Beste G, Schmidt FS, Stibora T, Skerra A. (1999) Proc Natl Acad Sci U S A. 96(5):1898-903; Gebauer and Skerra (2009) Curr Opinion in Chemical Biology 13:245-255). Anticalins possess an eight-stranded β-barrel which forms a highly conserved core unit among the lipocalins and naturally forms binding sites for ligands by means of four structurally variable loops at the open end. Anticalins, although not homologous to the IgG superfamily, show features that so far have been considered typical for the binding sites of antibodies: (i) high structural plasticity as a consequence of sequence variation and (ii) elevated conformational flexibility, allowing induced fit to targets with differing shape.

As used herein, the term "DARPin" refers to a designed ankyrin repeat domain (166 residues), which provides a rigid interface arising from typically three repeated β-turns. DARPins usually carry three repeats corresponding to an artificial consensus sequence, wherein six positions per repeat are randomised. Consequently, DARPins lack structural flexibility (Gebauer and Skerra, 2009).

The term "avimer", as used herein, refers to a class of antibody mimetics which consist of two or more peptide sequences of 30 to 35 amino acids each, which are derived from A-domains of various membrane receptors and which are connected by linker peptides. Binding of target molecules occurs via the A-domain and domains with the desired binding specificity can be selected, for example, by phage display techniques. The binding specificity of the different A-domains contained in an avimer may, but does not have to be identical (Weidle UH, et al., (2013), Cancer Genomics Proteomics; 10(4): 155-68).

A "nanofitin" (also known as affitin) is an antibody mimetic protein that is derived from the DNA binding protein Sac7d of Sulfolobus acidocaldarius. Nanofitins usually have a molecular weight of around 7kDa and are designed to specifically bind a target molecule by randomising the amino acids on the binding surface (Mouratou B, Béhar G, Paillard-Laurance L, Colinet S, Pecorari F., (2012) Methods Mol Biol.; 805:315-31).

The term "affilin", as used herein, refers to antibody mimetics that are developed by using either gamma-B crystalline or ubiquitin as a scaffold and modifying amino-acids on the surface of these proteins by random mutagenesis. Selection of affilins with the desired target specificity is effected, for example, by phage display or ribosome display techniques. Depending on the scaffold, affilins have a molecular weight of approximately 10 or 20kDa. As used herein, the term affilin also refers to di- or multimerised forms of affilins (Weidle UH, et al., (2013), Cancer Genomics Proteomics; 10(4):155-68).

A "Kunitz domain peptide" is derived from the Kunitz domain of a Kunitz-type protease inhibitor such as bovine pancreatic trypsin inhibitor (BPTI), amyloid precursor protein (APP) or tissue factor pathway inhibitor (TFPI). Kunitz domains have a molecular weight of approximately 6kDA and domains with the required target specificity can be selected by display techniques such as phage display (Weidle et al., (2013), Cancer Genomics Proteomics; 10(4):155-68).

As used herein, the term "Fynomer^{®}" refers to a non-immunoglobulin-derived binding polypeptide derived from the human Fyn SH3 domain. Fyn SH3-derived polypeptides are well-known in the art and have been described e.g. in Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12).

The term "trispecific binding molecule" as used herein refers to a polypeptide molecule that possesses three binding domains and is thus capable of binding, preferably specifically binding to three different epitopes. At least one of these three epitopes is an epitope of the protein of the fourth aspect of the invention. The two other epitopes may also be epitopes of the protein of the fourth aspect of the invention or may be epitopes of one or two different antigens. The trispecific binding molecule is preferably a TriTac. A TriTac is a T-cell engager for solid tumors which comprised of three binding domains being designed to have an extended serum half-life and be about one-third the size of a monoclonal antibody.

As used herein, the term "probody" refers to a protease-activatable antibody prodrug. A probody consists of an authentic IgG heavy chain and a modified light chain. A masking peptide is fused to the light chain through a peptide linker that is cleavable by tumor-specific proteases. The masking peptide prevents the probody binding to healthy tissues, thereby minimizing toxic side effects.

The present invention relates in a third aspect to a pharmaceutical composition comprising the compound of the first aspect or the conjugate of the second aspect.

In accordance with the present invention, the term "pharmaceutical composition" relates to a composition for administration to a patient, preferably a human patient. The pharmaceutical composition of the invention comprises the compounds recited above. It may, optionally, comprise further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day. The length of treatment needed to observe changes and the interval following treatment for responses to occur vary depending on the desired effect. The particular amounts may be determined by conventional tests which are well known to the person skilled in the art.

In accordance with a more preferred embodiment of the third aspect the pharmaceutical composition further comprises a pharmaceutically acceptable carrier, excipient or diluent.

Hence, the pharmaceutical composition of the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by well-known conventional methods.

The present invention relates in a fourth aspect to the compound, conjugate or pharmaceutical composition of any one of the preceding aspects for use as a medicament.

Regarding the use as a medicament it is to be understood that the diseases to be treated by the medicament is not particularly limited. The disease is preferably a disease that is mediated or at least in part mediated by the expression of LDH, preferably the overexpression of LDH. Examples are heart failure, hypothyroidism, anemia, and lung or liver disease.

The present invention relates in a fifth aspect to the compound, conjugate or the pharmaceutical composition of any of the preceding aspect for use in treating a cell proliferative disease.

The proliferative disease is a proliferative disease that is mediated or at least in part mediated by the expression of LDH, preferably the overexpression of LDH.

The cell proliferative disease can be a tumor and is preferably cancer. A tumor is an abnormal benign or malignant new growth of tissue that possesses no physiological function and arises from uncontrolled usually rapid cellular proliferation.

In accordance with a preferred embodiment of the fifth aspect the cell proliferative disease is cancer.

Cancer is an abnormal malignant new growth of tissue that possesses no physiological function and arises from uncontrolled usually rapid cellular proliferation.

The tumor or cancer is preferably a solid tumor or cancer. A solid tumor or cancer is an abnormal mass of tissue that usually does not contain cysts or liquid areas by contrast to a liquid tumor.

Many cancers show raised LDH levels. Also noncancerous conditions that can raise LDH levels include heart failure, hypothyroidism, anemia, and lung or liver disease.

The multiple roles of LDH in cancer are reviewed, for example, in Claps, Nature Reviews Clinical Oncology volume 19, pages749-762 (2022). High serum lactate dehydrogenase (LDH) levels are typically associated with a poor prognosis in many cancer types. LDH has a role in several aspects of oncogenesis, such as metabolic pathway plasticity and tumor cell immune evasion. Since LDH enzymes orchestrate various hallmarks of cancer they are a highly attractive targets for cancer therapy.

The capacity of X001, X089 and X091 to selectively impede the proliferation of cancerous cell lines without exerting excessive impact on the healthy cell line is shown in the appended examples.

In accordance with a more preferred embodiment of the fifth aspect the cancer is selected from breast cancer, ovarian cancer, endometrial cancer, vaginal cancer, vulva cancer, bladder cancer, salivary gland cancer, pancreatic cancer, thyroid cancer, kidney cancer, lung cancer, cancer concerning the upper gastrointestinal tract, colon cancer, colorectal cancer, prostate cancer, squamous-cell carcinoma of the head and neck, cervical cancer, glioblastomas, malignant ascites, lymphomas and leukemias.

The above list of cancers are non-limiting but preferred examples of cancers.

In accordance with another more preferred embodiment of the fifth aspect the cancer is a metastatic cancer.

Metastatic cancer is cancer that has spread from where it started to another part of the body. The original cancer is called the primary tumor. The cancer in another part of the body is called metastasis.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show.
**Figure 1****: Role of LDH in cancer metabolism and strategies to inhibition.** Cancer cells upregulate glycolysis and secrete lactate to the extracellular media. The isoform LDH5 is a key enzyme for this metabolic adaptation. Different population of cancer cells (oxidative) can use the secreted lactate as a source of energy, thanks to the isoform LDH1. Two main strategies have been described to inhibit LDHs: (i) traditional small molecules that compete with substrate/cofactor to bind to the active site, or (ii) allosteric inhibitors, most of them being peptides designed to target PPIs involved in the formation of active LDH.
**Figure 2****:** (A) Chemical structure of X001, X089 and X091. (B) Docking position of X001, X089 and X091 on LDHB and its key molecular interactions

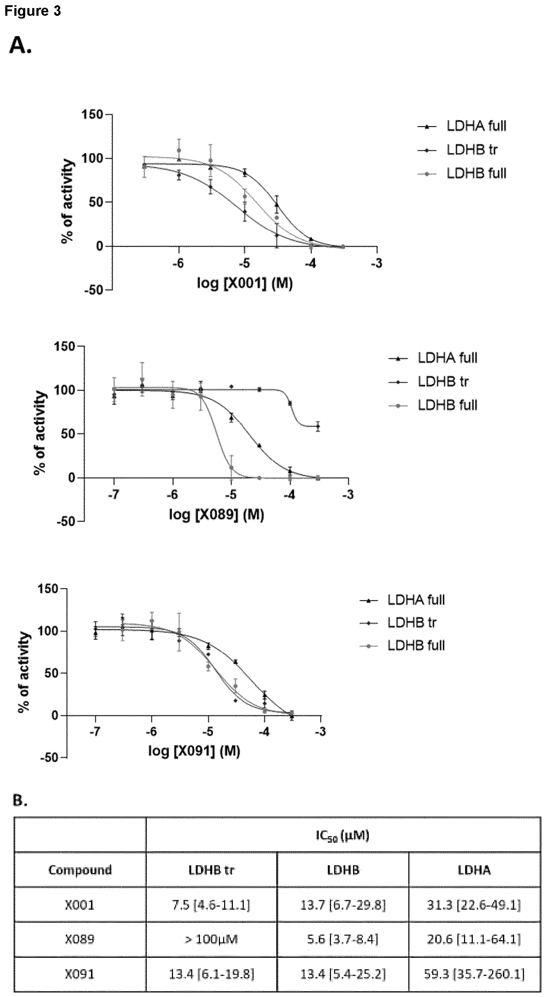

**Figure 3****:** (A) Dose-response curve of X001, X089 and X091 on LDHBtr, LDHB and LDHA. (B) LDHs inhibition (IC₅₀) of the three molecules. All experiments to determine IC₅₀ values were performed in triplicates at each compound dilution. Under bracket: 95% confidence interval.

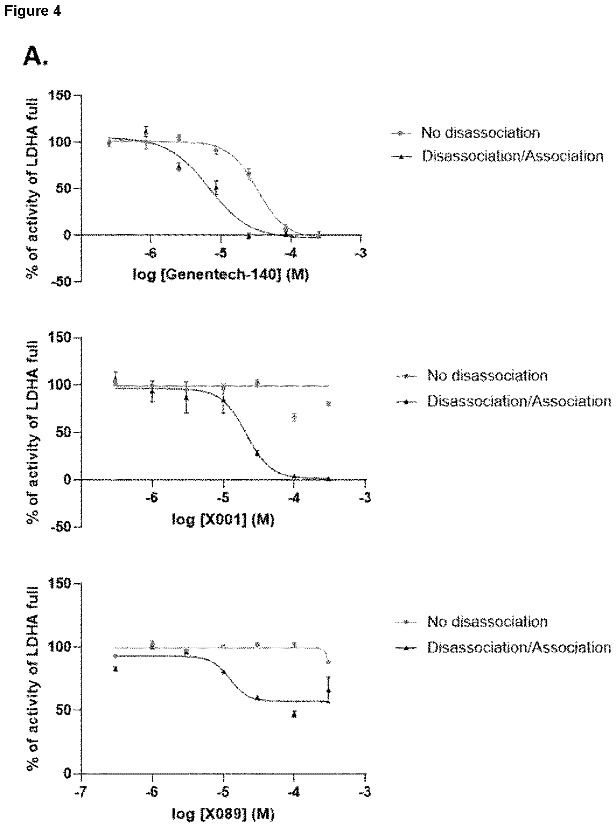

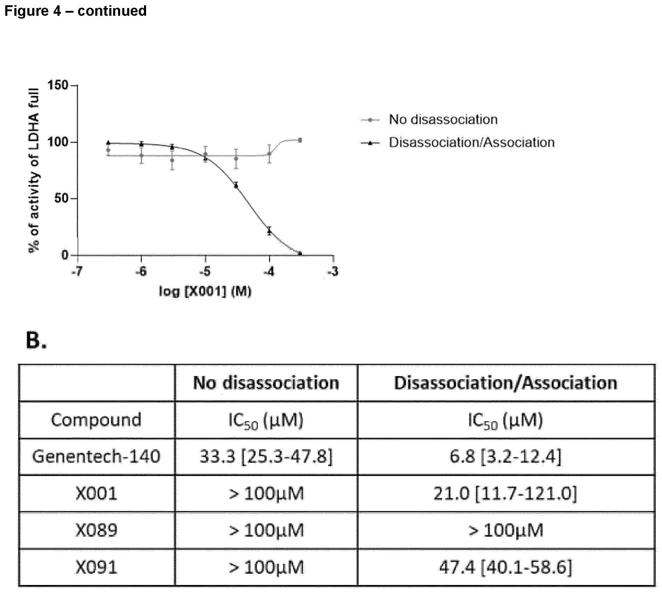

**Figure 4****:** (A) Dose-response curve of X001, X089, X091 and Genentech-140 on LDHA with or without disassociation/association. (B) LDHA inhibition (IC₅₀) of our identified hits and Genentech-140. All experiments to determine IC₅₀ values were performed in triplicates at each compound dilution. Under bracket: 95% confidence interval.

The examples illustrate the invention.

### Example 1 - Chemical synthesis of compounds X001, X089 and X091

### 1.1 Chemical synthesis of compound X001

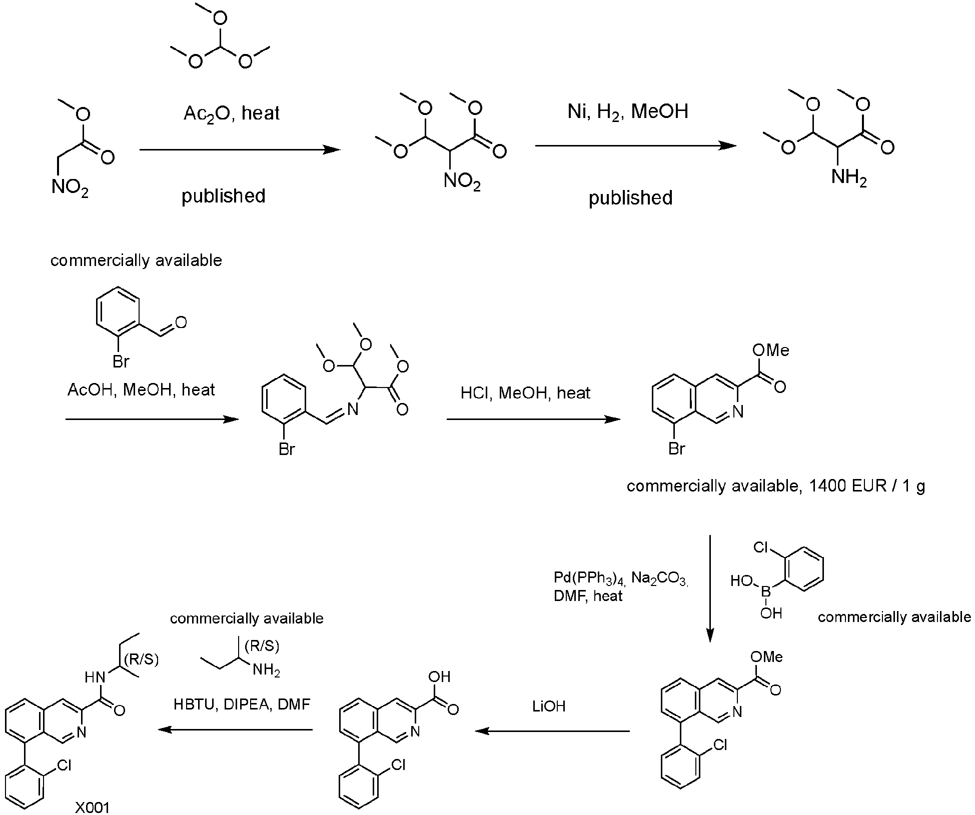

### 1.2. Chemical synthesis of compound X089

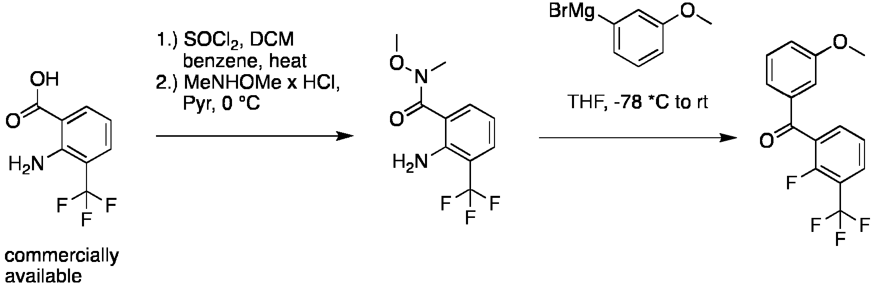

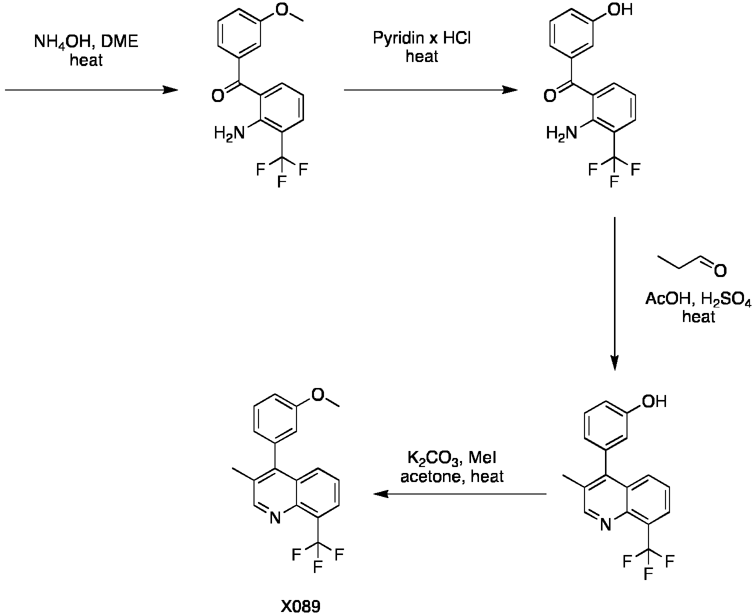

### 1.3. Chemical synthesis of compound X091

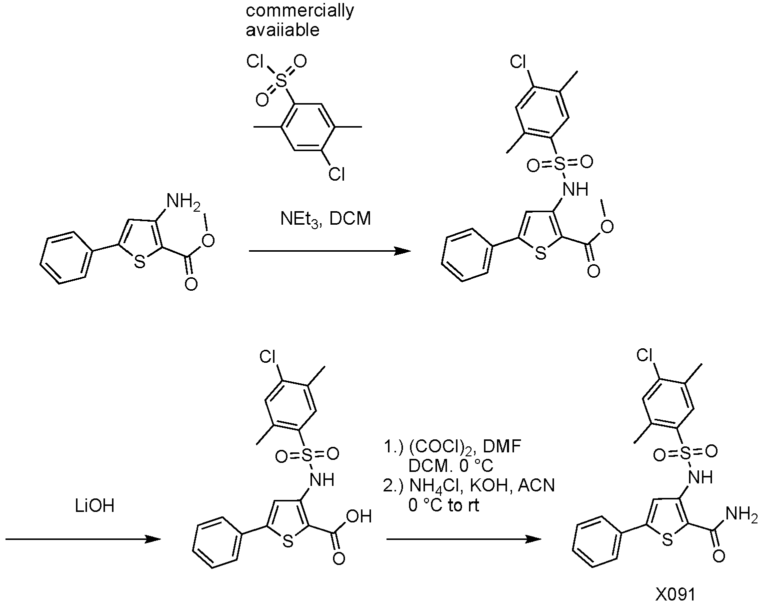

### Example 2 - Identification of compounds X001, X089 and X091 as inhibitors of LDH

### 2.1. AI assisted hit identification.

In this study, an in-house algorithm was employed for computational screening of small molecules to target the LDH tetramerization site, aiming to identify compounds capable of disrupting the enzyme's quaternary structure and consequently inhibiting its activity. The screening process involved molecular docking simulations and scoring functions to predict binding affinities. The Mcules internal library, comprising millions of compounds, served as the primary source for screening. Among the screened compounds, three molecules (MCULE-6217289136, MCULE-3439457046 and MCULE-6991677777), designated respectively as X001, X089 and X091 according to internal nomenclature, exhibited remarkable binding affinity to the LDH tetramerization site. Molecular docking simulations illustrated the binding orientation of the three molecules within the oligomerization site (Figure 2B), highlighting the key interactions with surrounding amino acids. This observation suggests the potential of these compounds to interfere with LDH tetramerization.

### 2.2. Biophysical and biochemical validation

To assess the validity of our primary virtual screening, nanoDSF was employed, a robust biophysical technique, to investigate the interaction of these 3 molecules with LDHs enzymes and their impact on enzymes stability. Following a protocol previously established in our lab⁴, LDHA, LDHB, and a truncated version of LDHB, which forms a weaker tetramer and is more susceptible to compounds inhibiting tetramerization (LDHBtr), were subjected to thermal denaturation assays in the presence of 100µM of X001, X089 and X091. The melting points of the proteins were then determined. Additionally, CB01, a binder previously identified by our lab with weak LDH inhibitory properties, was included as a positive control.

Our results, summarized in Table 2, reveal a significant effect of the three compounds on LDHBtr protein, with a decrease in melting point of -8.83°C for X091. This effect was also observed on LDHA and LDHB full length proteins. These findings seem to suggest that our hits interact with these proteins and potentially disrupting their stability.

**Table 2: Thermal shifts of the different compounds screened at 100 µM (n = 2).**

| | **LDHB tr** | | **LDHB** | | **LDHA** |
|---|---|---|---|---|---|
| **Compounds** | **ΔTm** (°**C**) | | | | |
| **CB01** | - *5,92* ± *0.10* | | - *1.92* ± *0.11* | | *-2.73* ± *0.10* |
| **X001** | | *- 0.51* ± *0.0* | - *0.4* ± *0.03* | | + *1.03* ± *0.05* |
| **X089** | | *+ 0.81* ± *0.35* | | - *1.4* ± *0.05* | + *0.17* ± *0.05* |
| **X091** | | - 8.83 ± *0.30* | *-3.8* ± *0.10* | | - *0.52* ± *0.21* |

To correlate the observed protein destabilization with potential inhibitory effects, IC₅₀ evaluations of X001, X089 and X091 were conducted on the same set of enzymes (Figure 3) using an assay previously developed in our lab.² Briefly, LDH activity was monitored through the conversion of pyruvate to lactate, accompanied by the consumption of the fluorescent cofactor, NADH.

Encouragingly, X001 and X091 demonstrated inhibitory activity against all three enzymes in these assays, with IC₅₀ values in the low micromolar range. X089 also demonstrated its ability to inhibit LDHA and LDHB full length in the micromolar range but without acting on LDHBtr maybe indicating a different mode of action.

To ascertain whether this inhibition stemmed from disruption of the tetrameric form of the proteins rather than conventional orthosteric or allosteric mechanisms, IC₅₀ evaluations were performed using a distinct enzymatic assay. This assay, inspired by literature reports, involved a dissociation-association LDH assay.⁸ Under acidic conditions (pH 2.5), LDHs partially dissociate and become inactive, but upon restoration of pH to 7.4, they reassemble into functionally active tetramers. LDHA activity was assessed after this dissociation-reassociation step in the presence or absence of X001, X089 and X091 but also with a classical orthosteric inhibitor (Genentech-140) as a negative control (Figure 4).

Notably, X001 and X091 demonstrated LDHA inhibition following the dissociation-reassociation process within the same range observed in our conventional enzymatic assay. However, when the enzyme was not dissociated, the inhibition was completely abolished. In contrast, the compound from Genentech maintained consistent inhibition in this assay, regardless of protein dissociation. For X089, no inhibition (before or after dissociation) could be observed using this assay, suggesting a different mode of action from the other two molecules and from the reference orthosteric inhibitor.

### 2.3. MST evaluation

Next the ability of our hits to interact with the LDH tetrameric interface was evaluated using LDHBtr. Their binding affinities (Kd) values with MicroScale Thermophoresis (MST) were determined experiments following a protocol previously described by our laboratory⁴ (Table 3).

**Table 3: Dissociation constant of the 3 compounds determined by Microscale thermophoresis (n = 3).**

| ***Compound*** | ***Kd on LDHB tr (µM)*** |
|---|---|
| ***X001*** | 4.20 ±1.91 |
| ***X089*** | 10.7 ± 2.2 |
| ***X091*** | > 100µM |

Interestingly, X001 and X089 depicted Kd values in the low micromolar range, comparable to the IC₅₀ previously determined, demonstrating their ability to bind and interact with this truncated version of the protein.

Taken together, these findings suggest that X001, and X091 affect LDHs' thermal stability and inhibit their activity by interacting at the tetrameric interface previously identified.

### 2.4. In cellulo evaluation

Finally, it was sought to validate the efficacy of our molecules by investigating their ability to inhibit the cellular proliferation of two distinct cancer cell lines, previously reported to be dependent on LDH activity¹: the oxidative MCF7 and the glycolytic MDA-MB-231, while sparing healthy BJ fibroblast proliferation. Cells were treated with two concentrations of X001, X089 and X091 (10X and 1X the enzymatic IC50), and the effects were monitored over a 72-hour period.

**Table 4: Cellular proliferation assay (Presto Blue readout / n = 3).**

| ***Compounds*** | ***10X IC50 (enzymatic)*** | | | ***1X IC50 (enzymatic)*** | | |
|---|---|---|---|---|---|---|
| | ***survival* % *after 72hrs*** | | | | | |
| | ***MCF7*** | ***MDA-MB-231*** | ***BJ fibroblast*** | ***MCF7*** | ***MDA-MB-231*** | ***BJ fibroblast*** |
| ***X001*** | 0 ± 0 | 0 ± 0 | 35.3 ± 17.4 | 91.9 ± 0.1 | 104.8 ± 6.5 | 70.7 ± 9.5 |
| ***X089*** | 4.3 ± 3.1 | 3.1 ± 1.5 | 45.5 ± 33.2 | 86.9 ± 10.9 | 69.5 ± 3.6 | 71.5 ± 37.5 |
| ***X091*** | 0 ± 0 | 0± 0 | 25.4 ± 12.7 | 53.7 ± 2.2 | 84.5 ± 7.6 | 45.8 ± 35.9 |

Results outlined in Table 4 demonstrate their capacity to selectively impede the proliferation of cancerous cell lines without exerting excessive impact on the healthy cell line. This validates the potential of our compound as a candidate for designing novel anticancer therapies targeting LDH inhibition through disruption of its tetrameric form. Moreover, it affirms the efficacy of this strategy and the virtual screening approach that was employed.

### Example 3 - In silico identification of further inhibitors of LDH based on compounds X001 and X091

The following workflow was used to determine based on compounds X001, X089 and X091 structurally related compounds that are also *bona fide* inhibitors of LDH:
- **Discovery Algorithm:** Our discovery algorithm, which uses docking simulations, identified the binding positions of compounds X001 and X091 and LDH.
- **Initial Structure Design:** Based on the docking simulation results the core structure of compounds X001 and X091 was proposed.
- **Side Chain Identification:** Then potential side chains for the variable groups were identified, ensuring that they would likely maintain or enhance binding affinity to LDH.
- **Combination Generation:** All possible combinations of these side chains with the core structure were generated.
- **Re-ranking and Optimization:** The thereby generated compounds were then re-ranked using the discovery algorithm as used in Example 2, considering factors such as energy optimization (e.g., docking scores) and structural similarities to the originally tested compounds X001 and X091.

**Table 5: Inhibitors of LDH as identified based on compounds X001 and X091 (IUPAC names)**

| | |
|---|---|
| (1) | N-(aminomethyl)-7-chloro-1-(2-chloro-6-hydroxyphenyl)-6-fluoroisoquinoline-3-carboxamide |
| (2) | N-(aminomethyl)-6,7-dichloro-1-(2-chloro-6-hydroxyphenyl)isoquinoline-3-carboxamide |
| (3) | 7-chloro-1-(2-chloro-6-hydroxyphenyl)-6-fluoro-N-[2-(methylamino)ethyl]isoquinoline-3-carboxamide |
| (4) | 7-chloro-1-(2-chloro-6-hydroxyphenyl)-6-fluoro-N-[3-(methylamino)propyl]isoquinoline-3-carboxamide |
| (5) | N-(2-aminoethyl)-7-chloro-1-(2-chloro-6-hydroxyphenyl)-6-fluoroisoquinoline-3-carboxamide |
| (6) | N-(3-aminopropyl)-7-chloro-1-(2-chloro-6-hydroxyphenyl)-6-fluoroisoquinoline-3-carboxamide |
| (7) | 7-chloro-1-(2-chloro-6-hydroxyphenyl)-6-fluoro-N-[(methylamino)methyl]isoquinoline-3-carboxamide |
| (8) | 6,7-dichloro-1-(2-chloro-6-hydroxyphenyl)-N-[3-(methylamino)propyl]isoquinoline-3-carboxamide |
| (9) | 7-chloro-1-[(2-chloro-6-hydroxyphenyl)methyl]-6-fluoro-N-[3-(methylamino)propyl]isoquinoline-3-carboxamide |
| (10) | 3-[3-(4-chloro-2,5-dimethylbenzenesulfonamido)-5-[4-(1-methylpiperidin-4-yl)phenyl]thiophen-2-yl]propanamide |
| (11) | 3-chloro-2-[7-chloro-6-fluoro-3-(piperazine-1-carbonyl)isoquinolin-1-yl]phenol |
| (12) | 6,7-dichloro-1-(2-chloro-6-hydroxyphenyl)-N-[2-(methylamino)ethyl]isoquinoline-3-carboxamide |
| (13) | 3-chloro-2-[7-chloro-6-fluoro-3-(4-methylpiperazine-1-carbonyl)isoquinolin-1-yl]phenol |
| (14) | N-(3-aminopropyl)-6,7-dichloro-1-(2-chloro-6-hydroxyphenyl)isoquinoline-3-carboxamide |
| (15) | 3-chloro-2-[6,7-dichloro-3-(4-methylpiperazine-1-carbonyl)isoquinolin-1-yl]phenol |
| (16) | N-(aminomethyl)-6,7-dichloro-1-[(2-chloro-6-hydroxyphenyl)methyl]isoquinoline-3-carboxamide |
| (17) | N-(2-aminoethyl)-1-(2-chloro-6-hydroxyphenyl)-6,7-difluoroisoquinoline-3-carboxamide |
| (18) | N-(2-aminoethyl)-6-chloro-1-(2-chloro-6-hydroxyphenyl)-7-fluoroisoquinoline-3-carboxamide |
| (19) | N-[2-(carbamoylmethyl)-5-[4-(piperidin-4-yl)phenyl]thiophen-3-yl]-4-chloro-2,5-dimethylbenzamide |
| (20) | 6-chloro-1-(2-chloro-6-hydroxyphenyl)-7-fluoro-N-[2-(methylamino)ethyl]isoquinoline-3-carboxamide |
| (21) | 3-chloro-2-[6,7-dichloro-3-(piperazine-1-carbonyl)isoquinolin-1-yl]phenol |
| (22) | N-(2-aminoethyl)-6,7-dichloro-1-(2-chloro-6-hydroxyphenyl)isoquinoline-3-carboxamide |
| (23) | 6-chloro-1-(2-chloro-6-hydroxyphenyl)-7-fluoro-N-[(methylamino)methyl]isoquinoline-3-carboxamide |
| (24) | 6,7-dichloro-1-(2-chloro-6-hydroxyphenyl)-N-[(methylamino)methyl]isoquinoline-3-carboxamide |
| (25) | 3-chloro-2-[6-chloro-7-fluoro-3-(piperazine-1-carbonyl)isoquinolin-1-yl]phenol |
| (26) | N-[2-(carbamoylmethyl)-5-[4-(1-methylpiperidin-4-yl)phenyl]thiophen-3-yl]-4-chloro-5-methoxy-2-methylbenzamide |
| (27) | 4-chloro-3-{2-[6,7-difluoro-3-(piperazine-1-carbonyl)isoquinolin-1-yl]ethyl}phenol |
| (28) | N-(3-aminopropyl)-6-chloro-1-(2-chloro-6-hydroxyphenyl)-7-fluoroisoquinoline-3-carboxamide |
| (29) | N-[2-(carbamoylmethyl)-5-[4-(piperidin-4-yl)phenyl]thiophen-3-yl]-4-chloro-5-hydroxy-2-methylbenzamide |
| (30) | N-(aminomethyl)-6-chloro-1-(2-chloro-6-hydroxyphenyl)-7-fluoroisoquinoline-3-carboxamide |
| (31) | N-(3-aminopropyl)-1-(2-chloro-6-hydroxyphenyl)-6,7-difluoroisoquinoline-3-carboxamide |
| (32) | 3-chloro-2-[6-chloro-7-fluoro-3-(4-methylpiperazine-1-carbonyl)isoquinolin-1-yl]phenol |
| (33) | 6-chloro-1-(2-chloro-6-hydroxyphenyl)-7-fluoro-N-[3-(methylamino)propyl]isoquinoline-3-carboxamide |
| (34) | 3-chloro-2-[6,7-difluoro-3-(piperazine-1-carbonyl)isoquinolin-1-yl]phenol |
| (35) | 3-[3-(4-chloro-5-methoxy-2-methylbenzenesulfonamido)-5-[4-(piperidin-4-yl)phenyl]thiophen-2-yl]propanamide |
| (36) | 3-chloro-2-{[6-chloro-7-fluoro-3-(piperazine-1-carbonyl)isoquinolin-1-yl]methyl}phenol |
| (37) | N-[2-(carbamoylmethyl)-5-[4-(piperidin-4-yl)phenyl]thiophen-3-yl]-4-chloro-5-methoxy-2-methylbenzamide |
| (38) | N-[2-(2-carbamoylethyl)-5-[4-(1-methylpiperidin-4-yl)phenyl]thiophen-3-yl]-4-chloro-5-hydroxy-2-methylbenzamide |
| (39) | 6-chloro-1-[(2-chloro-6-hydroxyphenyl)methyl]-7-fluoro-N-[(methylamino)methyl]isoquinoline-3-carboxamide |
| (40) | N-(aminomethyl)-1-[(2-chloro-6-hydroxyphenyl)methyl]-6,7-difluoroisoquinoline-3-carboxamide |
| (41) | 6-chloro-1-[(2-chloro-6-hydroxyphenyl)methyl]-7-fluoro-N-[2-(methylamino)ethyl]isoquinoline-3-carboxamide |
| (42) | N-[2-(carbamoylmethyl)-5-[4-(1-methylpiperidin-4-yl)phenyl]thiophen-3-yl]-4-chloro-5-hydroxy-2-methylbenzamide |
| (43) | N-(3-aminopropyl)-1-[(2-chloro-6-hydroxyphenyl)methyl]-6,7-difluoroisoquinoline-3-carboxamide |
| (44) | 6,7-dichloro-1-[(2-chloro-6-hydroxyphenyl)methyl]-N-[(methylamino)methyl]isoquinoline-3-carboxamide |
| (45) | N-(aminomethyl)-1-(2-chloro-6-hydroxyphenyl)-6,7-difluoroisoquinoline-3-carboxamide |
| (46) | 6-chloro-1-[(2-chloro-6-hydroxyphenyl)methyl]-7-fluoro-N-[3-(methylamino)propyl]isoquinoline-3-carboxamide |
| (47) | N-(aminomethyl)-6-chloro-1-[(2-chloro-6-hydroxyphenyl)methyl]-7-fluoroisoquinoline-3-carboxamide |
| (48) | 1-(2-chloro-6-hydroxyphenyl)-6,7-difluoro-N-[3-(methylamino)propyl]isoquinoline-3-carboxamide |
| (49) | 7-chloro-1-[(2-chloro-6-hydroxyphenyl)methyl]-6-fluoro-N-[(methylamino)methyl]isoquinoline-3-carboxamide |
| (50) | N-(aminomethyl)-7-chloro-1-[(2-chloro-6-hydroxyphenyl)methyl]-6-fluoroisoquinoline-3-carboxamide |
| (51) | 3-chloro-2-{[6-chloro-7-fluoro-3-(4-methylpiperazine-1-carbonyl)isoquinolin-1-yl]methyl}phenol |
| (52) | 1-[(2-chloro-6-hydroxyphenyl)methyl]-6,7-difluoro-N-[(methylamino)methyl]isoquinoline-3-carboxamide |
| (53) | 3-(4-chloro-2,5-dimethylbenzenesulfonamido)-5-[4-(piperidin-4-yl)phenyl]thiophene-2-carboxamide |
| (54) | 3-chloro-2-{[6,7-dichloro-3-(4-methylpiperazine-1-carbonyl)isoquinolin-1-yl]methyl}phenol |
| (55) | N-(3-aminopropyl)-6,7-dichloro-1-[(2-chloro-6-hydroxyphenyl)methyl]isoquinoline-3-carboxamide |
| (56) | 1-(2-chloro-6-hydroxyphenyl)-6,7-difluoro-N-[2-(methylamino)ethyl]isoquinoline-3-carboxamide |
| (57) | 3-chloro-2-[6,7-difluoro-3-(4-methylpiperazine-1-carbonyl)isoquinolin-1-yl]phenol |
| (58) | 1-(2-chloro-6-hydroxyphenyl)-6,7-difluoro-N-[(methylamino)methyl]isoquinoline-3-carboxamide |
| (59) | N-(2-aminoethyl)-1-[(2-chloro-6-hydroxyphenyl)methyl]-6,7-difluoroisoquinoline-3-carboxamide |
| (60) | 3-chloro-2-{[6,7-dichloro-3-(piperazine-1-carbonyl)isoquinolin-1-yl]methyl}phenol |
| (61) | N-(2-aminoethyl)-7-chloro-1-[(2-chloro-6-hydroxyphenyl)methyl]-6-fluoroisoquinoline-3-carboxamide |
| (62) | 2-[3-(4-chloro-5-methoxy-2-methylbenzenesulfonamido)-5-[4-(1-methylpiperidin-4-yl)phenyl]thiophen-2-yl]acetamide |
| (63) | 3-chloro-2-{[7-chloro-6-fluoro-3-(piperazine-1-carbonyl)isoquinolin-1-yl]methyl}phenol |
| (64) | 2-[3-(4-chloro-5-hydroxy-2-methylbenzenesulfonamido)-5-[4-(piperidin-4-yl)phenyl]thiophen-2-yl]acetamide |
| (65) | 3-chloro-2-{[6,7-difluoro-3-(piperazine-1-carbonyl)isoquinolin-1-yl]methyl}phenol |
| (66) | 3-chloro-2-{[7-chloro-6-fluoro-3-(4-methylpiperazine-1-carbonyl)isoquinolin-1-yl]methyl}phenol |
| (67) | 7-chloro-1-[(2-chloro-6-hydroxyphenyl)methyl]-6-fluoro-N-[2-(methylamino)ethyl]isoquinoline-3-carboxamide |
| (68) | 3-chloro-2-{[6,7-difluoro-3-(4-methylpiperazine-1-carbonyl)isoquinolin-1-yl]methyl}phenol |
| (69) | 4-chloro-3-{2-[7-chloro-6-fluoro-3-(4-methylpiperazine-1-carbonyl)isoquinolin-1-yl]ethyl}phenol |
| (70) | 6-chloro-1-[2-(2-chloro-5-hydroxyphenyl)ethyl]-7-fluoro-N-[3-(methylamino)propyl]isoquinoline-3-carboxamide |
| (71) | 1-[(2-chloro-6-hydroxyphenyl)methyl]-6,7-difluoro-N-[2-(methylamino)ethyl]isoquinoline-3-carboxamide |
| (72) | 3-[3-(4-chloro-5-hydroxy-2-methylbenzenesulfonamido)-5-[4-(piperidin-4-yl)phenyl]thiophen-2-yl]propanamide |
| (73) | 1-[(2-chloro-6-hydroxyphenyl)methyl]-6,7-difluoro-N-[3-(methylamino)propyl]isoquinoline-3-carboxamide |
| (74) | N-(aminomethyl)-7-chloro-1-[2-(2-chloro-5-hydroxyphenyl)ethyl]-6-fluoroisoquinoline-3-carboxamide |
| (75) | N-[2-(carbamoylmethyl)-5-phenylthiophen-3-yl]-4-chloro-5-hydroxy-2-methylbenzamide |
| (76) | N-[2-(2-carbamoylethyl)-5-[4-(piperidin-4-yl)phenyl]thiophen-3-yl]-4-chloro-2,5-dimethylbenzamide |
| (77) | 6,7-dichloro-1-[(2-chloro-6-hydroxyphenyl)methyl]-N-[3-(methylamino)propyl]isoquinoline-3-carboxamide |
| (78) | N-[2-(carbamoylmethyl)-5-[4-(1-methylpiperidin-4-yl)phenyl]thiophen-3-yl]-4-chloro-2,5-dimethylbenzamide |
| (79) | 4- chloro-3-{2-[6-chloro-7-fluoro-3-(4-methylpiperazine-1-carbonyl)isoquinolin-1-yl]ethyl}phenol |
| (80) | 3-[3-(4-chloro-2,5-dimethylbenzenesulfonamido)-5-[4-(piperidin-4-yl)phenyl]thiophen-2-yl]propanamide |
| (81) | 3-(4-chloro-5-methoxy-2-methylbenzenesulfonamido)-5-phenylthiophene-2-carboxamide |
| (82) | 3-[3-(4-chloro-5-hydroxy-2-methylbenzenesulfonamido)-5-[4-(1-methylpiperidin-4-yl)phenyl]thiophen-2-yl]propanamide |
| (83) | N-[2-(carbamoylmethyl)-5-phenylthiophen-3-yl]-4-chloro-2,5-dimethylbenzamide |
| (84) | N-[2-(2-carbamoylethyl)-5-[4-(1-methylpiperidin-4-yl)phenyl]thiophen-3-yl]-4-chloro-5-methoxy-2-methylbenzamide |
| (85) | 3-(4-chloro-2,5-dimethylbenzamido)-5-[4-(1-methylpiperidin-4-yl)phenyl]thiophene-2-carboxamide |
| (86) | 6-chloro-1-[2-(2-chloro-5-hydroxyphenyl)ethyl]-7-fluoro-N-[(methylamino)methyl]isoquinoline-3-carboxamide |
| (87) | 4-chloro-3-{2-[6,7-dichloro-3-(piperazine-1-carbonyl)isoquinolin-1-yl]ethyl}phenol |
| (88) | N-[2-(2-carbamoylethyl)-5-[4-(piperidin-4-yl)phenyl]thiophen-3-yl]-4-chloro-5-hydroxy-2-methylbenzamide |
| (89) | 6,7-dichloro-1-[2-(2-chloro-5-hydroxyphenyl)ethyl]-N-[2-(methylamino)ethyl]isoquinoline-3-carboxamide |
| (90) | 7-chloro-1-[2-(2-chloro-5-hydroxyphenyl)ethyl]-6-fluoro-N-[2-(methylamino)ethyl]isoquinoline-3-carboxamide |
| (91) | 2-[3-(4-chloro-5-methoxy-2-methylbenzenesulfonamido)-5-[4-(piperidin-4-yl)phenyl]thiophen-2-yl]acetamide |
| (92) | N-(2-aminoethyl)-6,7-dichloro-1-[(2-chloro-6-hydroxyphenyl)methyl]isoquinoline-3-carboxamide |
| (93) | N-[2-(carbamoylmethyl)-5-[4-(dimethylamino)butyl]thiophen-3-yl]-4-chloro-2,5-dimethylbenzamide |
| (94) | N-(3-aminopropyl)-6-chloro-1-[(2-chloro-6-hydroxyphenyl)methyl]-7-fluoroisoquinoline-3-carboxamide |
| (95) | N-(aminomethyl)-6-chloro-1-[2-(2-chloro-5-hydroxyphenyl)ethyl]-7-fluoroisoquinoline-3-carboxamide |
| (96) | N-(2-aminoethyl)-7-chloro-1-[2-(2-chloro-5-hydroxyphenyl)ethyl]-6-fluoroisoquinoline-3-carboxamide |
| (97) | 6,7-dichloro-1-[2-(2-chloro-5-hydroxyphenyl)ethyl]-N-[(methylamino)methyl]isoquinoline-3-carboxamide |
| (98) | N-(2-aminoethyl)-1-[2-(2-chloro-5-hydroxyphenyl)ethyl]-6,7-difluoroisoquinoline-3-carboxamide |
| (99) | 3-(4-chloro-5-methoxy-2-methylbenzamido)-5-[4-(1-methylpiperidin-4-yl)phenyl]thiophene-2-carboxamide |
| (100) | 1-[2-(2-chloro-5-hydroxyphenyl)ethyl]-6,7-difluoro-N-[2-(methylamino)ethyl]isoquinoline-3-carboxamide |
| (101) | N-(2-aminoethyl)-6-chloro-1-[(2-chloro-6-hydroxyphenyl)methyl]-7-fluoroisoquinoline-3-carboxamide |
| (102) | N-[2-(2-carbamoylethyl)-5-phenylthiophen-3-yl]-4-chloro-5-hydroxy-2-methylbenzamide |
| (103) | N-(aminomethyl)-1-[2-(2-chloro-5-hydroxyphenyl)ethyl]-6,7-difluoroisoquinoline-3-carboxamide |
| (104) | 7-chloro-1-[2-(2-chloro-5-hydroxyphenyl)ethyl]-6-fluoro-N-[3-(methylamino)propyl]isoquinoline-3-carboxamide |
| (105) | N-(3-aminopropyl)-7-chloro-1-[2-(2-chloro-5-hydroxyphenyl)ethyl]-6-fluoroisoquinoline-3-carboxamide |
| (106) | N-(2-aminoethyl)-6-chloro-1-[2-(2-chloro-5-hydroxyphenyl)ethyl]-7-fluoroisoquinoline-3-carboxamide |
| (107) | 1-[2-(2-chloro-5-hydroxyphenyl)ethyl]-6,7-difluoro-N-[3-(methylamino)propyl]isoquinoline-3-carboxamide |
| (108) | 4-chloro-3-{2-[6,7-difluoro-3-(4-methylpiperazine-1-carbonyl)isoquinolin-1-yl]ethyl}phenol |
| (109) | N-(3-aminopropyl)-6-chloro-1-[2-(2-chloro-5-hydroxyphenyl)ethyl]-7-fluoroisoquinoline-3-carboxamide |
| (110) | 1-[2-(2-chloro-5-hydroxyphenyl)ethyl]-6,7-difluoro-N-[(methylamino)methyl]isoquinoline-3-carboxamide |
| (111) | 4-chloro-3-{2-[6,7-dichloro-3-(4-methylpiperazine-1-carbonyl)isoquinolin-1-yl]ethyl}phenol |
| (112) | 3-[3-(4-chloro-5-methoxy-2-methylbenzenesulfonamido)-5-[4-(1-methylpiperidin-4-yl)phenyl]thiophen-2-yl]propanami |
| (113) | 2-[3-(4-chloro-2,5-dimethylbenzenesulfonamido)-5-[4-(piperidin-4-yl)phenyl]thiophen-2-yl]acetamide |
| (114) | 3-(4-chloro-2,5-dimethylbenzamido)-5-[4-(piperidin-4-yl)phenyl]thiophene-2-carboxamide |
| (115) | 6-chloro-1-[2-(2-chloro-5-hydroxyphenyl)ethyl]-7-fluoro-N-[2-(methylamino)ethyl]isoquinoline-3-carboxamide |
| (116) | N-(3-aminopropyl)-6,7-dichloro-1-[2-(2-chloro-5-hydroxyphenyl)ethyl]isoquinoline-3-carboxamide |
| (117) | N-[2-(2-carbamoylethyl)-5-[4-(1-methylpiperidin-4-yl)phenyl]thiophen-3-yl]-4-chloro-2,5-dimethylbenzamide |
| (118) | 3-(4-chloro-5-methoxy-2-methylbenzamido)-5-[4-(piperidin-4-yl)phenyl]thiophene-2-carboxamide |
| (119) | N-(2-aminoethyl)-6,7-dichloro-1-[2-(2-chloro-5-hydroxyphenyl)ethyl]isoquinoline-3-carboxamide |
| (120) | 2-[3-(4-chloro-5-hydroxy-2-methylbenzenesulfonamido)-5-phenylthiophen-2-yl]acetamide |
| (121) | 6,7-dichloro-1-[2-(2-chloro-5-hydroxyphenyl)ethyl]-N-[3-(methylamino)propyl]isoquinoline-3-carboxamide |
| (122) | 3-(4-chloro-5-hydroxy-2-methylbenzamido)-5-phenylthiophene-2-carboxamide |
| (123) | 3-(4-chloro-5-hydroxy-2-methylbenzenesulfonamido)-5-[4-(piperidin-4-yl)phenyl]thiophene-2-carboxamide |
| (124) | 6,7-dichloro-1-[(2-chloro-6-hydroxyphenyl)methyl]-N-[2-(methylamino)ethyl]isoquinoline-3-carboxamide |
| (125) | 3-(4-chloro-5-methoxy-2-methylbenzenesulfonamido)-5-[4-(1-methylpiperidin-4-yl)phenyl]thiophene-2-carboxamide |
| (126) | 2-[3-(4-chloro-5-hydroxy-2-methylbenzenesulfonamido)-5-[4-(1-methylpiperidin-4-yl)phenyl]thiophen-2-yl]acetamide |
| (127) | N-[2-(2-carbamoylethyl)-5-phenylthiophen-3-yl]-4-chloro-2,5-dimethylbenzamide |
| (128) | N-[2-(2-carbamoylethyl)-5-[2-(dimethylamino)ethyl]thiophen-3-yl]-4-chloro-5-hydroxy-2-methylbenzamide |
| (129) | 3-(4-chloro-5-methoxy-2-methylbenzamido)-5-phenylthiophene-2-carboxamide |
| (130) | N-(3-aminopropyl)-1-[2-(2-chloro-5-hydroxyphenyl)ethyl]-6,7-difluoroisoquinoline-3-carboxamide |
| (131) | 2-[3-(4-chloro-2,5-dimethylbenzenesulfonamido)-5-[4-(1-methylpiperidin-4-yl)phenyl]thiophen-2-yl]acetamide |
| (132) | 3-(4-chloro-5-hydroxy-2-methylbenzamido)-5-[4-(piperidin-4-yl)phenyl]thiophene-2-carboxamide |
| (133) | 7- chloro-1-[2-(2-chloro-5-hydroxyphenyl)ethyl]-6-fluoro-N-[(methylamino)methyl]isoquinoline-3-carboxamide |
| (134) | 4-chloro-3-{2-[7-chloro-6-fluoro-3-(piperazine-1-carbonyl)isoquinolin-1-yl]ethyl}phenol |
| (135) | N-[2-(2-carbamoylethyl)-5-[4-(piperidin-4-yl)phenyl]thiophen-3-yl]-4-chloro-5-methoxy-2-methylbenzamide |
| (136) | 3-(4-chloro-2,5-dimethylbenzenesulfonamido)-5-[4-(1-methylpiperidin-4-yl)phenyl]thiophene-2-carboxamide |
| (137) | N-[2-(2-carbamoylethyl)-5-[2-(dimethylamino)ethyl]thiophen-3-yl]-4-chloro-2,5-dimethylbenzamide |
| (138) | N-[2-(2-carbamoylethyl)-5-[3-(dimethylamino)propyl]thiophen-3-yl]-4-chloro-2,5-dimethylbenzamide |
| (139) | 3-(4-chloro-5-hydroxy-2-methylbenzamido)-5-[4-(1-methylpiperidin-4-yl)phenyl]thiophene-2-carboxamide |
| (140) | 2-[3-(4-chloro-5-hydroxy-2-methylbenzenesulfonamido)-5-[2-(dimethylamino)ethyl]thiophen-2-yl]acetamide |
| (141) | 2-[3-(4-chloro-5-hydroxy-2-methylbenzenesulfonamido)-5-[3-(dimethylamino)propyl]thiophen-2-yl]acetamide |
| (142) | N-[2-(carbamoylmethyl)-5-[3-(dimethylamino)propyl]thiophen-3-yl]-4-chloro-2,5-dimethylbenzamide |
| (143) | 3-(4-chloro-5-hydroxy-2-methylbenzenesulfonamido)-5-[4-(dimethylamino)butyl]thiophene-2-carboxamide |
| (144) | 3-(4-chloro-2,5-dimethylbenzenesulfonamido)-5-phenylthiophene-2-carboxamide |
| (145) | 3-[3-(4-chloro-2,5-dimethylbenzenesulfonamido)-5-phenylthiophen-2-yl]propanamide |
| (146) | N-[2-(2-carbamoylethyl)-5-[3-(dimethylamino)propyl]thiophen-3-yl]-4-chloro-5-methoxy-2-methylbenzamide |
| (147) | 3-(4-chloro-5-hydroxy-2-methylbenzamido)-5-[4-(dimethylamino)butyl]thiophene-2-carboxamide |
| (148) | 2-[3-(4-chloro-5-hydroxy-2-methylbenzenesulfonamido)-5-[4-(dimethylamino)butyl]thiophen-2-yl]acetamide |
| (149) | 3-(4-chloro-5-hydroxy-2-methylbenzamido)-5-[3-(dimethylamino)propyl]thiophene-2-carboxamide |
| (150) | 3-(4-chloro-2,5-dimethylbenzamido)-5-[2-(dimethylamino)ethyl]thiophene-2-carboxamide |
| (151) | N-(aminomethyl)-6,7-dichloro-1-[2-(2-chloro-5-hydroxyphenyl)ethyl]isoquinoline-3-carboxamide |
| (152) | 3-(4-chloro-2,5-dimethylbenzamido)-5-[3-(dimethylamino)propyl]thiophene-2-carboxamide |
| (153) | 3-(4-chloro-5-hydroxy-2-methylbenzenesulfonamido)-5-[2-(dimethylamino)ethyl]thiophene-2-carboxamide |
| (154) | N-[2-(2-carbamoylethyl)-5-[4-(dimethylamino)butyl]thiophen-3-yl]-4-chloro-2,5-dimethylbenzamide |
| (155) | 3-(4-chloro-5-hydroxy-2-methylbenzamido)-5-[2-(dimethylamino)ethyl]thiophene-2-carboxamide |
| (156) | 3-[3-(4-chloro-2,5-dimethylbenzenesulfonamido)-5-[2-(dimethylamino)ethyl]thiophen-2-yl]propanamide |
| (157) | 3-[3-(4-chloro-5-methoxy-2-methylbenzenesulfonamido)-5-[3-(dimethylamino)propyl]thiophen-2-yl]propanamide |
| (158) | 3-(4-chloro-2,5-dimethylbenzenesulfonamido)-5-[3-(dimethylamino)propyl]thiophene-2-carboxamide |
| (159) | N-[2-(carbamoylmethyl)-5-[3-(dimethylamino)propyl]thiophen-3-yl]-4-chloro-5-hydroxy-2-methylbenzamide |
| (160) | 3-(4-chloro-5-hydroxy-2-methylbenzenesulfonamido)-5-phenylthiophene-2-carboxamide |
| (161) | 3-[3-(4-chloro-5-methoxy-2-methylbenzenesulfonamido)-5-[4-(dimethylamino)butyl]thiophen-2-yl]propanamide |
| (162) | N-[2-(carbamoylmethyl)-5-[2-(dimethylamino)ethyl]thiophen-3-yl]-4-chloro-2,5-dimethylbenzamide |
| (163) | N-[2-(2-carbamoylethyl)-5-[2-(dimethylamino)ethyl]thiophen-3-yl]-4-chloro-5-methoxy-2-methylbenzamide |
| (164) | 3-(4-chloro-2,5-dimethylbenzenesulfonamido)-5-[4-(dimethylamino)butyl]thiophene-2-carboxamide |
| (165) | 2-[3-(4-chloro-2,5-dimethylbenzenesulfonamido)-5-[2-(dimethylamino)ethyl]thiophen-2-yl]acetamide |
| (166) | 3-[3-(4-chloro-5-methoxy-2-methylbenzenesulfonamido)-5-[2-(dimethylamino)ethyl]thiophen-2-yl]propanamide |
| (167) | N-(3-aminopropyl)-7-chloro-1-[(2-chloro-6-hydroxyphenyl)methyl]-6-fluoroisoquinoline-3-carboxamide |
| (168) | N-[2-(carbamoylmethyl)-5-[2-(dimethylamino)ethyl]thiophen-3-yl]-4-chloro-5-methoxy-2-methylbenzamide |
| (169) | N-[2-(2-carbamoylethyl)-5-phenylthiophen-3-yl]-4-chloro-5-methoxy-2-methylbenzamide |
| (170) | 3-(4-chloro-2,5-dimethylbenzamido)-5-[4-(dimethylamino)butyl]thiophene-2-carboxamide |
| (171) | N-[2-(carbamoylmethyl)-5-phenylthiophen-3-yl]-4-chloro-5-methoxy-2-methylbenzamide |
| (172) | 3-[3-(4-chloro-5-hydroxy-2-methylbenzenesulfonamido)-5-[2-(dimethylamino)ethyl]thiophen-2-yl]propanamide |
| (173) | N-[2-(2-carbamoylethyl)-5-[4-(dimethylamino)butyl]thiophen-3-yl]-4-chloro-5-hydroxy-2-methylbenzamide |
| (174) | N-[2-(carbamoylmethyl)-5-[2-(dimethylamino)ethyl]thiophen-3-yl]-4-chloro-5-hydroxy-2-methylbenzamide |
| (175) | 3-[3-(4-chloro-2,5-dimethylbenzenesulfonamido)-5-[4-(dimethylamino)butyl]thiophen-2-yl]propanamide |
| (176) | 3-(4-chloro-2,5-dimethylbenzenesulfonamido)-5-[2-(dimethylamino)ethyl]thiophene-2-carboxamide |
| (177) | 2-[3-(4-chloro-2,5-dimethylbenzenesulfonamido)-5-[4-(dimethylamino)butyl]thiophen-2-yl]acetamide |
| (178) | 3-[3-(4-chloro-2,5-dimethylbenzenesulfonamido)-5-[3-(dimethylamino)propyl]thiophen-2-yl]propanamide |
| (179) | N-[2-(carbamoylmethyl)-5-[4-(dimethylamino)butyl]thiophen-3-yl]-4-chloro-5-methoxy-2-methylbenzamide |
| (180) | N-[2-(carbamoylmethyl)-5-[3-(dimethylamino)propyl]thiophen-3-yl]-4-chloro-5-methoxy-2-methylbenzamide |
| (181) | 3-(4-chloro-2,5-dimethylbenzamido)-5-phenylthiophene-2-carboxamide |
| (182) | 3-(4-chloro-5-methoxy-2-methylbenzamido)-5-[2-(dimethylamino)ethyl]thiophene-2-carboxamide |
| (183) | 2-[3-(4-chloro-2,5-dimethylbenzenesulfonamido)-5-phenylthiophen-2-yl]acetamide |
| (184) | N-[2-(2-carbamoylethyl)-5-[3-(dimethylamino)propyl]thiophen-3-yl]-4-chloro-5-hydroxy-2-methylbenzamide |
| (185) | 3-[3-(4-chloro-5-hydroxy-2-methylbenzenesulfonamido)-5-phenylthiophen-2-yl]propanamide |
| (186) | 3-(4-chloro-5-methoxy-2-methylbenzenesulfonamido)-5-[3-(dimethylamino)propyl]thiophene-2-carboxamide |
| (187) | 2-[3-(4-chloro-2,5-dimethylbenzenesulfonamido)-5-[3-(dimethylamino)propyl]thiophen-2-yl]acetamide |
| (188) | N-[2-(carbamoylmethyl)-5-[4-(dimethylamino)butyl]thiophen-3-yl]-4-chloro-5-hydroxy-2-methylbenzamide |
| (189) | 3-[3-(4-chloro-5-methoxy-2-methylbenzenesulfonamido)-5-phenylthiophen-2-yl]propanamide |
| (190) | N-[2-(2-carbamoylethyl)-5-[4-(dimethylamino)butyl]thiophen-3-yl]-4-chloro-5-methoxy-2-methylbenzamide |
| (191) | 4- chloro-3-{2-[6-chloro-7-fluoro-3-(piperazine-1-carbonyl)isoquinolin-1-yl]ethyl}phenol |

**Table 6: Inhibitors of LDH as identified based on compounds X001 and X091 (SMILES names)**

| | |
|---|---|
| (1) | C1(C3=C(Cl)C=CC=C3O)=C(C=C(Cl)C(F)=C2)C2=CC(C(NCN)=O)=N1 |
| (2) | C1(C3=C(Cl)C=CC=C3O)=C(C=C(Cl)C(Cl)=C2)C2=CC(C(NCN)=O)=N1 |
| (3) | C1(C3=C(Cl)C=CC=C3O)=C(C=C(Cl)C(F)=C2)C2=CC(C(NCCNC)=O)=N1 |
| (4) | C1(C3=C(Cl)C=CC=C3O)=C(C=C(Cl)C(F)=C2)C2=CC(C(NCCCNC)=O)=N1 |
| (5) | C1(C3=C(Cl)C=CC=C3O)=C(C=C(Cl)C(F)=C2)C2=CC(C(NCCN)=O)=N1 |
| (6) | C1(C3=C(Cl)C=CC=C3O)=C(C=C(Cl)C(F)=C2)C2=CC(C(NCCCN)=O)=N1 |
| (7) | C1(C3=C(Cl)C=CC=C3O)=C(C=C(Cl)C(F)=C2)C2=CC(C(NCNC)=O)=N1 |
| (8) | C1(C3=C(Cl)C=CC=C3O)=C(C=C(Cl)C(Cl)=C2)C2=CC(C(NCCCNC)=O)=N1 |
| (9) | C1(CC3=C(Cl)C=CC=C3O)=C(C=C(Cl)C(F)=C2)C2=CC(C(NCCCNC)=O)=N1 |
| (10) | C1(C3=CC=C(C4CCN(C)CC4)C=C3)=CC(NS(=O)(=O)C2=C(C)C=C(Cl)C(C)=C2)=C(CCC(=O)N)S1 |
| (11) | C1(C3=C(Cl)C=CC=C3O)=C(C=C(Cl)C(F)=C2)C2=CC(C(N4CCNCC4)=O)=N1 |
| (12) | C1(C3=C(Cl)C=CC=C3O)=C(C=C(Cl)C(Cl)=C2)C2=CC(C(NCCNC)=O)=N1 |
| (13) | C1(C3=C(Cl)C=CC=C3O)=C(C=C(Cl)C(F)=C2)C2=CC(C(N4CCN(C)CC4)=O)=N1 |
| (14) | C1(C3=C(Cl)C=CC=C3O)=C(C=C(Cl)C(Cl)=C2)C2=CC(C(NCCCN)=O)=N1 |
| (15) | C1(C3=C(Cl)C=CC=C3O)=C(C=C(Cl)C(Cl)=C2)C2=CC(C(N4CCN(C)CC4)=O)=N1 |
| (16) | C1(CC3=C(Cl)C=CC=C3O)=C(C=C(Cl)C(Cl)=C2)C2=CC(C(NCN)=O)=N1 |
| (17) | C1(C3=C(Cl)C=CC=C3O)=C(C=C(F)C(F)=C2)C2=CC(C(NCCN)=O)=N1 |
| (18) | C1(C3=C(Cl)C=CC=C3O)=C(C=C(F)C(Cl)=C2)C2=CC(C(NCCN)=O)=N1 |
| (19) | C1(C3=CC=C(C4CCNCC4)C=C3)=CC(NC(=O)C2=C(C)C=C(Cl)C(C)=C2)=C(CC(=O)N)S1 |
| (20) | C1(C3=C(Cl)C=CC=C3O)=C(C=C(F)C(Cl)=C2)C2=CC(C(NCCNC)=O)=N1 |
| (21) | C1(C3=C(Cl)C=CC=C3O)=C(C=C(Cl)C(Cl)=C2)C2=CC(C(N4CCNCC4)=O)=N1 |
| (22) | C1(C3=C(Cl)C=CC=C3O)=C(C=C(Cl)C(Cl)=C2)C2=CC(C(NCCN)=O)=N1 |
| (23) | C1(C3=C(Cl)C=CC=C3O)=C(C=C(F)C(Cl)=C2)C2=CC(C(NCNC)=O)=N1 |
| (24) | C1(C3=C(Cl)C=CC=C3O)=C(C=C(Cl)C(Cl)=C2)C2=CC(C(NCNC)=O)=N1 |
| (25) | C1(C3=C(Cl)C=CC=C3O)=C(C=C(F)C(Cl)=C2)C2=CC(C(N4CCNCC4)=O)=N1 |
| (26) | C1(C3=CC=C(C4CCN(C)CC4)C=C3)=CC(NC(=O)C2=C(C)C=C(Cl)C(OC)=C2)=C(CC(=O)N)S1 |
| (27) | C1(CCC3=CC(O)=CC=C3Cl)=C(C=C(F)C(F)=C2)C2=CC(C(N4CCNCC4)=O)=N1 |
| (28) | C1(C3=C(Cl)C=CC=C3O)=C(C=C(F)C(Cl)=C2)C2=CC(C(NCCCN)=O)=N1 |
| (29) | C1(C3=CC=C(C4CCNCC4)C=C3)=CC(NC(=O)C2=C(C)C=C(Cl)C(O)=C2)=C(CC(=O)N)S1 |
| (30) | C1(C3=C(Cl)C=CC=C3O)=C(C=C(F)C(Cl)=C2)C2=CC(C(NCN)=O)=N1 |
| (31) | C1(C3=C(Cl)C=CC=C3O)=C(C=C(F)C(F)=C2)C2=CC(C(NCCCN)=O)=N1 |
| (32) | C1(C3=C(Cl)C=CC=C3O)=C(C=C(F)C(Cl)=C2)C2=CC(C(N4CCN(C)CC4)=O)=N1 |
| (33) | C1(C3=C(Cl)C=CC=C3O)=C(C=C(F)C(Cl)=C2)C2=CC(C(NCCCNC)=O)=N1 |
| (34) | C1(C3=C(Cl)C=CC=C3O)=C(C=C(F)C(F)=C2)C2=CC(C(N4CCNCC4)=O)=N1 |
| (35) | C1(C3=CC=C(C4CCNCC4)C=C3)=CC(NS(=O)(=O)C2=C(C)C=C(Cl)C(OC)=C2)=C(CCC(=O)N)S1 |
| (36) | C1(CC3=C(Cl)C=CC=C3O)=C(C=C(F)C(Cl)=C2)C2=CC(C(N4CCNCC4)=O)=N1 |
| (37) | C1(C3=CC=C(C4CCNCC4)C=C3)=CC(NC(=O)C2=C(C)C=C(Cl)C(OC)=C2)=C(CC(=O)N)S1 |
| (38) | C1(C3=CC=C(C4CCN(C)CC4)C=C3)=CC(NC(=O)C2=C(C)C=C(Cl)C(O)=C2)=C(CCC(=O)N)S1 |
| (39) | C1(CC3=C(Cl)C=CC=C3O)=C(C=C(F)C(Cl)=C2)C2=CC(C(NCNC)=O)=N1 |
| (40) | C1(CC3=C(Cl)C=CC=C3O)=C(C=C(F)C(F)=C2)C2=CC(C(NCN)=O)=N1 |
| (41) | C1(CC3=C(Cl)C=CC=C3O)=C(C=C(F)C(Cl)=C2)C2=CC(C(NCCNC)=O)=N1 |
| (42) | C1(C3=CC=C(C4CCN(C)CC4)C=C3)=CC(NC(=O)C2=C(C)C=C(Cl)C(O)=C2)=C(CC(=O)N)S1 |
| (43) | C1(CC3=C(Cl)C=CC=C3O)=C(C=C(F)C(F)=C2)C2=CC(C(NCCCN)=O)=N1 |
| (44) | C1(CC3=C(Cl)C=CC=C3O)=C(C=C(Cl)C(Cl)=C2)C2=CC(C(NCNC)=O)=N1 |
| (45) | C1(C3=C(Cl)C=CC=C3O)=C(C=C(F)C(F)=C2)C2=CC(C(NCN)=O)=N1 |
| (46) | C1(CC3=C(Cl)C=CC=C3O)=C(C=C(F)C(Cl)=C2)C2=CC(C(NCCCNC)=O)=N1 |
| (47) | C1(CC3=C(Cl)C=CC=C3O)=C(C=C(F)C(Cl)=C2)C2=CC(C(NCN)=O)=N1 |
| (48) | C1(C3=C(Cl)C=CC=C3O)=C(C=C(F)C(F)=C2)C2=CC(C(NCCCNC)=O)=N1 |
| (49) | C1(CC3=C(Cl)C=CC=C3O)=C(C=C(Cl)C(F)=C2)C2=CC(C(NCNC)=O)=N1 |
| (50) | C1(CC3=C(Cl)C=CC=C3O)=C(C=C(Cl)C(F)=C2)C2=CC(C(NCN)=O)=N1 |
| (51) | C1(CC3=C(Cl)C=CC=C3O)=C(C=C(F)C(Cl)=C2)C2=CC(C(N4CCN(C)CC4)=O)=N1 |
| (52) | C1(CC3=C(Cl)C=CC=C3O)=C(C=C(F)C(F)=C2)C2=CC(C(NCNC)=O)=N1 |
| (53) | C1(C3=CC=C(C4CCNCC4)C=C3)=CC(NS(=O)(=O)C2=C(C)C=C(Cl)C(C)=C2)=C(C(=O)N)S1 |
| (54) | C1(CC3=C(Cl)C=CC=C3O)=C(C=C(Cl)C(Cl)=C2)C2=CC(C(N4CCN(C)CC4)=O)=N1 |
| (55) | C1(CC3=C(Cl)C=CC=C3O)=C(C=C(Cl)C(Cl)=C2)C2=CC(C(NCCCN)=O)=N1 |
| (56) | C1(C3=C(Cl)C=CC=C3O)=C(C=C(F)C(F)=C2)C2=CC(C(NCCNC)=O)=N1 |
| (57) | C1(C3=C(Cl)C=CC=C3O)=C(C=C(F)C(F)=C2)C2=CC(C(N4CCN(C)CC4)=O)=N1 |
| (58) | C1(C3=C(Cl)C=CC=C3O)=C(C=C(F)C(F)=C2)C2=CC(C(NCNC)=O)=N1 |
| (59) | C1(CC3=C(Cl)C=CC=C3O)=C(C=C(F)C(F)=C2)C2=CC(C(NCCN)=O)=N1 |
| (60) | C1(CC3=C(Cl)C=CC=C3O)=C(C=C(Cl)C(Cl)=C2)C2=CC(C(N4CCNCC4)=O)=N1 |
| (61) | C1(CC3=C(Cl)C=CC=C3O)=C(C=C(Cl)C(F)=C2)C2=CC(C(NCCN)=O)=N1 |
| (62) | C1(C3=CC=C(C4CCN(C)CC4)C=C3)=CC(NS(=O)(=O)C2=C(C)C=C(Cl)C(OC)=C2)=C(CC(=O)N)S1 |
| (63) | C1(CC3=C(Cl)C=CC=C3O)=C(C=C(Cl)C(F)=C2)C2=CC(C(N4CCNCC4)=O)=N1 |
| (64) | C1(C3=CC=C(C4CCNCC4)C=C3)=CC(NS(=O)(=O)C2=C(C)C=C(Cl)C(O)=C2)=C(CC(=O)N)S1 |
| (65) | C1(CC3=C(Cl)C=CC=C3O)=C(C=C(F)C(F)=C2)C2=CC(C(N4CCNCC4)=O)=N1 |
| (66) | C1(CC3=C(Cl)C=CC=C3O)=C(C=C(Cl)C(F)=C2)C2=CC(C(N4CCN(C)CC4)=O)=N1 |
| (67) | C1(CC3=C(Cl)C=CC=C3O)=C(C=C(Cl)C(F)=C2)C2=CC(C(NCCNC)=O)=N1 |
| (68) | C1(CC3=C(Cl)C=CC=C3O)=C(C=C(F)C(F)=C2)C2=CC(C(N4CCN(C)CC4)=O)=N1 |
| (69) | C1(CCC3=CC(O)=CC=C3Cl)=C(C=C(Cl)C(F)=C2)C2=CC(C(N4CCN(C)CC4)=O)=N1 |
| (70) | C1(CCC3=CC(O)=CC=C3Cl)=C(C=C(F)C(Cl)=C2)C2=CC(C(NCCCNC)=O)=N1 |
| (71) | C1(CC3=C(Cl)C=CC=C3O)=C(C=C(F)C(F)=C2)C2=CC(C(NCCNC)=O)=N1 |
| (72) | C1(C3=CC=C(C4CCNCC4)C=C3)=CC(NS(=O)(=O)C2=C(C)C=C(Cl)C(O)=C2)=C(CCC(=O)N)S1 |
| (73) | C1(CC3=C(Cl)C=CC=C3O)=C(C=C(F)C(F)=C2)C2=CC(C(NCCCNC)=O)=N1 |
| (74) | C1(CCC3=CC(O)=CC=C3Cl)=C(C=C(Cl)C(F)=C2)C2=CC(C(NCN)=O)=N1 |
| (75) | C1(C3=CC=CC=C3)=CC(NC(=O)C2=C(C)C=C(Cl)C(O)=C2)=C(CC(=O)N)S1 |
| (76) | C1(C3=CC=C(C4CCNCC4)C=C3)=CC(NC(=O)C2=C(C)C=C(Cl)C(C)=C2)=C(CCC(=O)N)S1 |
| (77) | C1(CC3=C(Cl)C=CC=C3O)=C(C=C(Cl)C(Cl)=C2)C2=CC(C(NCCCNC)=O)=N1 |
| (78) | C1(C3=CC=C(C4CCN(C)CC4)C=C3)=CC(NC(=O)C2=C(C)C=C(Cl)C(C)=C2)=C(CC(=O)N)S1 |
| (79) | C1(CCC3=CC(O)=CC=C3Cl)=C(C=C(F)C(Cl)=C2)C2=CC(C(N4CCN(C)CC4)=O)=N1 |
| (80) | C1(C3=CC=C(C4CCNCC4)C=C3)=CC(NS(=O)(=O)C2=C(C)C=C(CI)C(C)=C2)=C(CCC(=O)N)S1 |
| (81) | C1(C3=CC=CC=C3)=CC(NS(=O)(=O)C2=C(C)C=C(Cl)C(OC)=C2)=C(C(=O)N)S1 |
| (82) | C1(C3=CC=C(C4CCN(C)CC4)C=C3)=CC(NS(=O)(=O)C2=C(C)C=C(Cl)C(O)=C2)=C(CCC(=O)N)S1 |
| (83) | C1(C3=CC=CC=C3)=CC(NC(=O)C2=C(C)C=C(Cl)C(C)=C2)=C(CC(=O)N)S1 |
| (84) | C1(C3=CC=C(C4CCN(C)CC4)C=C3)=CC(NC(=O)C2=C(C)C=C(Cl)C(OC)=C2)=C(CCC(=O)N)S1 |
| (85) | C1(C3=CC=C(C4CCN(C)CC4)C=C3)=CC(NC(=O)C2=C(C)C=C(Cl)C(C)=C2)=C(C(=O)N)S1 |
| (86) | C1(CCC3=CC(O)=CC=C3Cl)=C(C=C(F)C(Cl)=C2)C2=CC(C(NCNC)=O)=N1 |
| (87) | C1(CCC3=CC(O)=CC=C3Cl)=C(C=C(Cl)C(Cl)=C2)C2=CC(C(N4CCNCC4)=O)=N1 |
| (88) | C1(C3=CC=C(C4CCNCC4)C=C3)=CC(NC(=O)C2=C(C)C=C(Cl)C(O)=C2)=C(CCC(=O)N)S1 |
| (89) | C1(CCC3=CC(O)=CC=C3Cl)=C(C=C(Cl)C(Cl)=C2)C2=CC(C(NCCNC)=O)=N1 |
| (90) | C1(CCC3=CC(O)=CC=C3Cl)=C(C=C(Cl)C(F)=C2)C2=CC(C(NCCNC)=O)=N1 |
| (91) | C1(C3=CC=C(C4CCNCC4)C=C3)=CC(NS(=O)(=O)C2=C(C)C=C(Cl)C(OC)=C2)=C(CC(=O)N)S1 |
| (92) | C1(CC3=C(Cl)C=CC=C3O)=C(C=C(Cl)C(Cl)=C2)C2=CC(C(NCCN)=O)=N1 |
| (93) | C1(CCCCN(C)C)=CC(NC(=O)C2=C(C)C=C(Cl)C(C)=C2)=C(CC(=O)N)S1 |
| (94) | C1(CC3=C(Cl)C=CC=C3O)=C(C=C(F)C(Cl)=C2)C2=CC(C(NCCCN)=O)=N1 |
| (95) | C1(CCC3=CC(O)=CC=C3Cl)=C(C=C(F)C(Cl)=C2)C2=CC(C(NCN)=O)=N1 |
| (96) | C1(CCC3=CC(O)=CC=C3Cl)=C(C=C(Cl)C(F)=C2)C2=CC(C(NCCN)=O)=N1 |
| (97) | C1(CCC3=CC(O)=CC=C3Cl)=C(C=C(Cl)C(Cl)=C2)C2=CC(C(NCNC)=O)=N1 |
| (98) | C1(CCC3=CC(O)=CC=C3Cl)=C(C=C(F)C(F)=C2)C2=CC(C(NCCN)=O)=N1 |
| (99) | C1(C3=CC=C(C4CCN(C)CC4)C=C3)=CC(NC(=O)C2=C(C)C=C(Cl)C(OC)=C2)=C(C(=O)N)S1 |
| (100) | C1(CCC3=CC(O)=CC=C3Cl)=C(C=C(F)C(F)=C2)C2=CC(C(NCCNC)=O)=N1 |
| (101) | C1(CC3=C(Cl)C=CC=C3O)=C(C=C(F)C(Cl)=C2)C2=CC(C(NCCN)=O)=N1 |
| (102) | C1(C3=CC=CC=C3)=CC(NC(=O)C2=C(C)C=C(Cl)C(O)=C2)=C(CCC(=O)N)S1 |
| (103) | C1(CCC3=CC(O)=CC=C3Cl)=C(C=C(F)C(F)=C2)C2=CC(C(NCN)=O)=N1 |
| (104) | C1(CCC3=CC(O)=CC=C3Cl)=C(C=C(Cl)C(F)=C2)C2=CC(C(NCCCNC)=O)=N1 |
| (105) | C1(CCC3=CC(O)=CC=C3Cl)=C(C=C(Cl)C(F)=C2)C2=CC(C(NCCCN)=O)=N1 |
| (106) | C1(CCC3=CC(O)=CC=C3Cl)=C(C=C(F)C(Cl)=C2)C2=CC(C(NCCN)=O)=N1 |
| (107) | C1(CCC3=CC(O)=CC=C3Cl)=C(C=C(F)C(F)=C2)C2=CC(C(NCCCNC)=O)=N1 |
| (108) | C1(CCC3=CC(O)=CC=C3Cl)=C(C=C(F)C(F)=C2)C2=CC(C(N4CCN(C)CC4)=O)=N1 |
| (109) | C1(CCC3=CC(O)=CC=C3Cl)=C(C=C(F)C(Cl)=C2)C2=CC(C(NCCCN)=O)=N1 |
| (110) | C1(CCC3=CC(O)=CC=C3Cl)=C(C=C(F)C(F)=C2)C2=CC(C(NCNC)=O)=N1 |
| (111) | C1(CCC3=CC(O)=CC=C3Cl)=C(C=C(Cl)C(Cl)=C2)C2=CC(C(N4CCN(C)CC4)=O)=N1 |
| (112) | C1(C3=CC=C(C4CCN(C)CC4)C=C3)=CC(NS(=O)(=O)C2=C(C)C=C(Cl)C(OC)=C2)=C(CCC(=O)N)S1 |
| (113) | C1(C3=CC=C(C4CCNCC4)C=C3)=CC(NS(=O)(=O)C2=C(C)C=C(Cl)C(C)=C2)=C(CC(=O)N)S1 |
| (114) | C1(C3=CC=C(C4CCNCC4)C=C3)=CC(NC(=O)C2=C(C)C=C(CI)C(C)=C2)=C(C(=O)N)S1 |
| (115) | C1(CCC3=CC(O)=CC=C3Cl)=C(C=C(F)C(Cl)=C2)C2=CC(C(NCCNC)=O)=N1 |
| (116) | C1(CCC3=CC(O)=CC=C3Cl)=C(C=C(Cl)C(Cl)=C2)C2=CC(C(NCCCN)=O)=N1 |
| (117) | C1(C3=CC=C(C4CCN(C)CC4)C=C3)=CC(NC(=O)C2=C(C)C=C(Cl)C(C)=C2)=C(CCC(=O)N)S1 |
| (118) | C1(C3=CC=C(C4CCNCC4)C=C3)=CC(NC(=O)C2=C(C)C=C(Cl)C(OC)=C2)=C(C(=O)N)S1 |
| (119) | C1(CCC3=CC(O)=CC=C3Cl)=C(C=C(Cl)C(Cl)=C2)C2=CC(C(NCCN)=O)=N1 |
| (120) | C1(C3=CC=CC=C3)=CC(NS(=O)(=O)C2=C(C)C=C(Cl)C(O)=C2)=C(CC(=O)N)S1 |
| (121) | C1(CCC3=CC(O)=CC=C3Cl)=C(C=C(Cl)C(Cl)=C2)C2=CC(C(NCCCNC)=O)=N1 |
| (122) | C1(C3=CC=CC=C3)=CC(NC(=O)C2=C(C)C=C(Cl)C(O)=C2)=C(C(=O)N)S1 |
| (123) | C1(C3=CC=C(C4CCNCC4)C=C3)=CC(NS(=O)(=O)C2=C(C)C=C(Cl)C(O)=C2)=C(C(=O)N)S1 |
| (124) | C1(CC3=C(Cl)C=CC=C3O)=C(C=C(Cl)C(Cl)=C2)C2=CC(C(NCCNC)=O)=N1 |
| (125) | C1(C3=CC=C(C4CCN(C)CC4)C=C3)=CC(NS(=O)(=O)C2=C(C)C=C(Cl)C(OC)=C2)=C(C(=O)N)S1 |
| (126) | C1(C3=CC=C(C4CCN(C)CC4)C=C3)=CC(NS(=O)(=O)C2=C(C)C=C(Cl)C(O)=C2)=C(CC(=O)N)S1 |
| (127) | C1(C3=CC=CC=C3)=CC(NC(=O)C2=C(C)C=C(CI)C(C)=C2)=C(CCC(=O)N)S1 |
| (128) | C1(CCN(C)C)=CC(NC(=O)C2=C(C)C=C(Cl)C(O)=C2)=C(CCC(=O)N)S1 |
| (129) | C1(C3=CC=CC=C3)=CC(NC(=O)C2=C(C)C=C(CI)C(OC)=C2)=C(C(=O)N)S1 |
| (130) | C1(CCC3=CC(O)=CC=C3Cl)=C(C=C(F)C(F)=C2)C2=CC(C(NCCCN)=O)=N1 |
| (131) | C1(C3=CC=C(C4CCN(C)CC4)C=C3)=CC(NS(=O)(=O)C2=C(C)C=C(Cl)C(C)=C2)=C(CC(=O)N)S1 |
| (132) | C1(C3=CC=C(C4CCNCC4)C=C3)=CC(NC(=O)C2=C(C)C=C(Cl)C(O)=C2)=C(C(=O)N)S1 |
| (133) | C1(CCC3=CC(O)=CC=C3Cl)=C(C=C(Cl)C(F)=C2)C2=CC(C(NCNC)=O)=N1 |
| (134) | C1(CCC3=CC(O)=CC=C3Cl)=C(C=C(Cl)C(F)=C2)C2=CC(C(N4CCNCC4)=O)=N1 |
| (135) | C1(C3=CC=C(C4CCNCC4)C=C3)=CC(NC(=O)C2=C(C)C=C(Cl)C(OC)=C2)=C(CCC(=O)N)S1 |
| (136) | C1(C3=CC=C(C4CCN(C)CC4)C=C3)=CC(NS(=O)(=O)C2=C(C)C=C(Cl)C(C)=C2)=C(C(=O)N)S1 |
| (137) | C1(CCN(C)C)=CC(NC(=O)C2=C(C)C=C(Cl)C(C)=C2)=C(CCC(=O)N)S1 |
| (138) | C1(CCCN(C)C)=CC(NC(=O)C2=C(C)C=C(Cl)C(C)=C2)=C(CCC(=O)N)S1 |
| (139) | C1(C3=CC=C(C4CCN(C)CC4)C=C3)=CC(NC(=O)C2=C(C)C=C(Cl)C(O)=C2)=C(C(=O)N)S1 |
| (140) | C1(CCN(C)C)=CC(NS(=O)(=O)C2=C(C)C=C(Cl)C(O)=C2)=C(CC(=O)N)S1 |
| (141) | C1(CCCN(C)C)=CC(NS(=O)(=O)C2=C(C)C=C(Cl)C(O)=C2)=C(CC(=O)N)S1 |
| (142) | C1(CCCN(C)C)=CC(NC(=O)C2=C(C)C=C(Cl)C(C)=C2)=C(CC(=O)N)S1 |
| (143) | C1(CCCCN(C)C)=CC(NS(=O)(=O)C2=C(C)C=C(Cl)C(O)=C2)=C(C(=O)N)S1 |
| (144) | C1(C3=CC=CC=C3)=CC(NS(=O)(=O)C2=C(C)C=C(Cl)C(C)=C2)=C(C(=O)N)S1 |
| (145) | C1(C3=CC=CC=C3)=CC(NS(=O)(=O)C2=C(C)C=C(Cl)C(C)=C2)=C(CCC(=O)N)S1 |
| (146) | C1(CCCN(C)C)=CC(NC(=O)C2=C(C)C=C(Cl)C(OC)=C2)=C(CCC(=O)N)S1 |
| (147) | C1(CCCCN(C)C)=CC(NC(=O)C2=C(C)C=C(Cl)C(O)=C2)=C(C(=O)N)S1 |
| (148) | C1(CCCCN(C)C)=CC(NS(=O)(=O)C2=C(C)C=C(Cl)C(O)=C2)=C(CC(=O)N)S1 |
| (149) | C1(CCCN(C)C)=CC(NC(=O)C2=C(C)C=C(Cl)C(O)=C2)=C(C(=O)N)S1 |
| (150) | C1(CCN(C)C)=CC(NC(=O)C2=C(C)C=C(Cl)C(C)=C2)=C(C(=O)N)S1 |
| (151) | C1(CCC3=CC(O)=CC=C3Cl)=C(C=C(Cl)C(Cl)=C2)C2=CC(C(NCN)=O)=N1 |
| (152) | C1(CCCN(C)C)=CC(NC(=O)C2=C(C)C=C(Cl)C(C)=C2)=C(C(=O)N)S1 |
| (153) | C1(CCN(C)C)=CC(NS(=O)(=O)C2=C(C)C=C(Cl)C(O)=C2)=C(C(=O)N)S1 |
| (154) | C1(CCCCN(C)C)=CC(NC(=O)C2=C(C)C=C(Cl)C(C)=C2)=C(CCC(=O)N)S1 |
| (155) | C1(CCN(C)C)=CC(NC(=O)C2=C(C)C=C(Cl)C(O)=C2)=C(C(=O)N)S1 |
| (156) | C1(CCN(C)C)=CC(NS(=O)(=O)C2=C(C)C=C(Cl)C(C)=C2)=C(CCC(=O)N)S1 |
| (157) | C1(CCCN(C)C)=CC(NS(=O)(=O)C2=C(C)C=C(Cl)C(OC)=C2)=C(CCC(=O)N)S1 |
| (158) | C1(CCCN(C)C)=CC(NS(=O)(=O)C2=C(C)C=C(Cl)C(C)=C2)=C(C(=O)N)S1 |
| (159) | C1(CCCN(C)C)=CC(NC(=O)C2=C(C)C=C(Cl)C(O)=C2)=C(CC(=O)N)S1 |
| (160) | C1(C3=CC=CC=C3)=CC(NS(=O)(=O)C2=C(C)C=C(Cl)C(O)=C2)=C(C(=O)N)S1 |
| (161) | C1(CCCCN(C)C)=CC(NS(=O)(=O)C2=C(C)C=C(Cl)C(OC)=C2)=C(CCC(=O)N)S1 |
| (162) | C1(CCN(C)C)=CC(NC(=O)C2=C(C)C=C(Cl)C(C)=C2)=C(CC(=O)N)S1 |
| (163) | C1(CCN(C)C)=CC(NC(=O)C2=C(C)C=C(Cl)C(OC)=C2)=C(CCC(=O)N)S1 |
| (164) | C1(CCCCN(C)C)=CC(NS(=O)(=O)C2=C(C)C=C(Cl)C(C)=C2)=C(C(=O)N)S1 |
| (165) | C1(CCN(C)C)=CC(NS(=O)(=O)C2=C(C)C=C(Cl)C(C)=C2)=C(CC(=O)N)S1 |
| (166) | C1(CCN(C)C)=CC(NS(=O)(=O)C2=C(C)C=C(Cl)C(OC)=C2)=C(CCC(=O)N)S1 |
| (167) | C1(CC3=C(Cl)C=CC=C3O)=C(C=C(Cl)C(F)=C2)C2=CC(C(NCCCN)=O)=N1 |
| (168) | C1(CCN(C)C)=CC(NC(=O)C2=C(C)C=C(Cl)C(OC)=C2)=C(CC(=O)N)S1 |
| (169) | C1(C3=CC=CC=C3)=CC(NC(=O)C2=C(C)C=C(Cl)C(OC)=C2)=C(CCC(=O)N)S1 |
| (170) | C1(CCCCN(C)C)=CC(NC(=O)C2=C(C)C=C(Cl)C(C)=C2)=C(C(=O)N)S1 |
| (171) | C1(C3=CC=CC=C3)=CC(NC(=O)C2=C(C)C=C(Cl)C(OC)=C2)=C(CC(=O)N)S1 |
| (172) | C1(CCN(C)C)=CC(NS(=O)(=O)C2=C(C)C=C(Cl)C(O)=C2)=C(CCC(=O)N)S1 |
| (173) | C1(CCCCN(C)C)=CC(NC(=O)C2=C(C)C=C(Cl)C(O)=C2)=C(CCC(=O)N)S1 |
| (174) | C1(CCN(C)C)=CC(NC(=O)C2=C(C)C=C(Cl)C(O)=C2)=C(CC(=O)N)S1 |
| (175) | C1(CCCCN(C)C)=CC(NS(=O)(=O)C2=C(C)C=C(Cl)C(C)=C2)=C(CCC(=O)N)S1 |
| (176) | C1(CCN(C)C)=CC(NS(=O)(=O)C2=C(C)C=C(Cl)C(C)=C2)=C(C(=O)N)S1 |
| (177) | C1(CCCCN(C)C)=CC(NS(=O)(=O)C2=C(C)C=C(Cl)C(C)=C2)=C(CC(=O)N)S1 |
| (178) | C1(CCCN(C)C)=CC(NS(=O)(=O)C2=C(C)C=C(Cl)C(C)=C2)=C(CCC(=O)N)S1 |
| (179) | C1(CCCCN(C)C)=CC(NC(=O)C2=C(C)C=C(Cl)C(OC)=C2)=C(CC(=O)N)S1 |
| (180) | C1(CCCN(C)C)=CC(NC(=O)C2=C(C)C=C(Cl)C(OC)=C2)=C(CC(=O)N)S1 |
| (181) | C1(C3=CC=CC=C3)=CC(NC(=O)C2=C(C)C=C(Cl)C(C)=C2)=C(C(=O)N)S1 |
| (182) | C1(CCN(C)C)=CC(NC(=O)C2=C(C)C=C(Cl)C(OC)=C2)=C(C(=O)N)S1 |
| (183) | C1(C3=CC=CC=C3)=CC(NS(=O)(=O)C2=C(C)C=C(Cl)C(C)=C2)=C(CC(=O)N)S1 |
| (184) | C1(CCCN(C)C)=CC(NC(=O)C2=C(C)C=C(Cl)C(O)=C2)=C(CCC(=O)N)S1 |
| (185) | C1(C3=CC=CC=C3)=CC(NS(=O)(=O)C2=C(C)C=C(Cl)C(O)=C2)=C(CCC(=O)N)S1 |
| (186) | C1(CCCN(C)C)=CC(NS(=O)(=O)C2=C(C)C=C(Cl)C(OC)=C2)=C(C(=O)N)S1 |
| (187) | C1(CCCN(C)C)=CC(NS(=O)(=O)C2=C(C)C=C(Cl)C(C)=C2)=C(CC(=O)N)S1 |
| (188) | C1(CCCCN(C)C)=CC(NC(=O)C2=C(C)C=C(Cl)C(O)=C2)=C(CC(=O)N)S1 |
| (189) | C1(C3=CC=CC=C3)=CC(NS(=O)(=O)C2=C(C)C=C(Cl)C(OC)=C2)=C(CCC(=O)N)S1 |
| (190) | C1(CCCCN(C)C)=CC(NC(=O)C2=C(C)C=C(Cl)C(OC)=C2)=C(CCC(=O)N)S1 |
| (191) | C1(CCC3=CC(O)=CC=C3Cl)=C(C=C(F)C(Cl)=C2)C2=CC(C(N4CCNCC4)=O)=N1 |

**Table 7: The 191 Inhibitors of LDH according to the numbering in Tables 5 and 6 ("Rank") classified into clusters (cluster_id), and as being derived from X001 and X091 (Root compounds)**

| **Rank** | **cluster_id** | **Root compounds** |
|---|---|---|
| 1 | 2 | 001 |
| 2 | 2 | 001 |
| 3 | 0 | 001 |
| 4 | 0 | 001 |
| 5 | 2 | 001 |
| 6 | 2 | 001 |
| 7 | 0 | 001 |
| 8 | 0 | 001 |
| 9 | 5 | 001 |
| 10 | 13 | 091 |
| 11 | 1 | 001 |
| 12 | 0 | 001 |
| 13 | 1 | 001 |
| 14 | 2 | 001 |
| 15 | 1 | 001 |
| 16 | 6 | 001 |
| 17 | 2 | 001 |
| 18 | 2 | 001 |
| 19 | 3 | 091 |
| 20 | 0 | 001 |
| 21 | 1 | 001 |
| 22 | 2 | 001 |
| 23 | 0 | 001 |
| 24 | 0 | 001 |
| 25 | 1 | 001 |
| 26 | 11 | 091 |
| 27 | 8 | 001 |
| 28 | 2 | 001 |
| 29 | 3 | 091 |
| 30 | 2 | 001 |
| 31 | 2 | 001 |
| 32 | 1 | 001 |
| 33 | 0 | 001 |
| 34 | 1 | 001 |
| 35 | 13 | 091 |
| 36 | 4 | 001 |
| 37 | 3 | 091 |
| 38 | 12 | 091 |
| 39 | 5 | 001 |
| 40 | 6 | 001 |
| 41 | 5 | 001 |
| 42 | 3 | 091 |
| 43 | 6 | 001 |
| 44 | 5 | 001 |
| 45 | 2 | 001 |
| 46 | 5 | 001 |
| 47 | 6 | 001 |
| 48 | 0 | 001 |
| 49 | 5 | 001 |
| 50 | 6 | 001 |
| 51 | 4 | 001 |
| 52 | 5 | 001 |
| 53 | 16 | 091 |
| 54 | 4 | 001 |
| 55 | 6 | 001 |
| 56 | 0 | 001 |
| 57 | 1 | 001 |
| 58 | 0 | 001 |
| 59 | 6 | 001 |
| 60 | 4 | 001 |
| 61 | 6 | 001 |
| 62 | 13 | 091 |
| 63 | 4 | 001 |
| 64 | 7 | 091 |
| 65 | 4 | 001 |
| 66 | 4 | 001 |
| 67 | 5 | 001 |
| 68 | 4 | 001 |
| 69 | 8 | 001 |
| 70 | 9 | 001 |
| 71 | 5 | 001 |
| 72 | 7 | 091 |
| 73 | 5 | 001 |
| 74 | 10 | 001 |
| 75 | 12 | 091 |
| 76 | 3 | 091 |
| 77 | 5 | 001 |
| 78 | 3 | 091 |
| 79 | 8 | 001 |
| 80 | 7 | 091 |
| 81 | 16 | 091 |
| 82 | 13 | 091 |
| 83 | 12 | 091 |
| 84 | 11 | 091 |
| 85 | 11 | 091 |
| 86 | 9 | 001 |
| 87 | 8 | 001 |
| 88 | 3 | 091 |
| 89 | 9 | 001 |
| 90 | 9 | 001 |
| 91 | 7 | 091 |
| 92 | 6 | 001 |
| 93 | 15 | 091 |
| 94 | 6 | 001 |
| 95 | 10 | 001 |
| 96 | 10 | 001 |
| 97 | 9 | 001 |
| 98 | 10 | 001 |
| 99 | 11 | 091 |
| 100 | 9 | 001 |
| 101 | 6 | 001 |
| 102 | 12 | 091 |
| 103 | 10 | 001 |
| 104 | 9 | 001 |
| 105 | 10 | 001 |
| 106 | 10 | 001 |
| 107 | 9 | 001 |
| 108 | 8 | 001 |
| 109 | 10 | 001 |
| 110 | 9 | 001 |
| 111 | 8 | 001 |
| 112 | 13 | 091 |
| 113 | 7 | 091 |
| 114 | 11 | 091 |
| 115 | 9 | 001 |
| 116 | 10 | 001 |
| 117 | 13 | 091 |
| 118 | 11 | 091 |
| 119 | 10 | 001 |
| 120 | 7 | 091 |
| 121 | 9 | 001 |
| 122 | 12 | 091 |
| 123 | 7 | 091 |
| 124 | 5 | 001 |
| 125 | 13 | 091 |
| 126 | 7 | 091 |
| 127 | 12 | 091 |
| 128 | 14 | 091 |
| 129 | 11 | 091 |
| 130 | 10 | 001 |
| 131 | 13 | 091 |
| 132 | 3 | 091 |
| 133 | 9 | 001 |
| 134 | 8 | 001 |
| 135 | 3 | 091 |
| 136 | 16 | 091 |
| 137 | 14 | 091 |
| 138 | 14 | 091 |
| 139 | 11 | 091 |
| 140 | 19 | 091 |
| 141 | 19 | 091 |
| 142 | 15 | 091 |
| 143 | 18 | 091 |
| 144 | 16 | 091 |
| 145 | 16 | 091 |
| 146 | 14 | 091 |
| 147 | 17 | 091 |
| 148 | 19 | 091 |
| 149 | 17 | 091 |
| 150 | 17 | 091 |
| 151 | 10 | 001 |
| 152 | 17 | 091 |
| 153 | 18 | 091 |
| 154 | 14 | 091 |
| 155 | 17 | 091 |
| 156 | 19 | 091 |
| 157 | 19 | 091 |
| 158 | 18 | 091 |
| 159 | 15 | 091 |
| 160 | 16 | 091 |
| 161 | 19 | 091 |
| 162 | 15 | 091 |
| 163 | 14 | 091 |
| 164 | 18 | 091 |
| 165 | 19 | 091 |
| 166 | 19 | 091 |
| 167 | 6 | 001 |
| 168 | 15 | 091 |
| 169 | 12 | 091 |
| 170 | 17 | 091 |
| 171 | 12 | 091 |
| 172 | 19 | 091 |
| 173 | 14 | 091 |
| 174 | 15 | 091 |
| 175 | 19 | 091 |
| 176 | 18 | 091 |
| 177 | 19 | 091 |
| 178 | 19 | 091 |
| 179 | 15 | 091 |
| 180 | 15 | 091 |
| 181 | 16 | 091 |
| 182 | 17 | 091 |
| 183 | 16 | 091 |
| 184 | 14 | 091 |
| 185 | 12 | 091 |
| 186 | 18 | 091 |
| 187 | 19 | 091 |
| 188 | 15 | 091 |
| 189 | 13 | 091 |
| 190 | 14 | 091 |
| 191 | 8 | 001 |

It is of note that the *in silico* identification of further inhibitors of LDH based on compounds X089 did not reveal further inhibitors of LDH based on X089.

### Example 4 - Materials and methods

All cell lines were from ATCC. All reagents were purchased from chemical suppliers and used without purification. Unless stated otherwise, all chemicals were from Sigma-Aldrich.

### 4.1. NanoDSF

NanoDSF assays were performed as described previously.⁴ Briefly, solutions of proteins (LDH-1, LDH-5 or LDH-Htr) were evaluated on a Prometheus device (NanoTemper Technologies), at 5 µM with and without 100 µM of compounds. Evaluations were run in a 50 mM sodium phosphate and 100 mM NaCl pH 7.6 buffer with 2% DMSO. According to standard manufacturer's procedures, samples were poured into capillaries and heated from 27°C up to 95°C (1°C/min) while following fluorescence emission at 330 and 350 nm. Melting temperatures were extracted from the derivative of the 350/330 nm fluorescence ratios upon increasing temperature. Compounds showing stabilization or destabilization of the protein higher than 1°C were selected for an enzymatic evaluation.

### 4.2. Enzymatic assay

### a. IC₅₀ evaluation

The IC₅₀ evaluation was carried out following a reported procedure.⁴ LDH(s) activity was measured in 96-well plates (96 µL per well) at 37 °C by monitoring NADH fluorescence (Ex 340 nM/Em 460 nM) over time with a SpectraMax m2e spectrophotometer (Molecular Devices) after addition of different compound concentrations (5% DMSO final concentration). Assays were performed in LDH(s) assay buffer (50 mM Tris, pH 8.0, and 100 mM NaCl). Substrates and enzyme concentrations were as follows: pyruvate, 3 mM; NADH , 300 µM; LDH-1 and LDH-5, 10 nM LDH-Htr, 200 nM.

### b. Association/dissociation assay

The ability of the most promising compounds to disrupt LDH oligomeric state was evaluated following a reported procedure.⁸ Briefly, LDH-5 was diluted to 900 nM in 0.01 M phosphate buffer (pH 7.4), 1 mM EDTA, and 0.05% BSA. Five microliters of protein solution were transferred into a Nunc 96- well polypropylene microplate (#249944) and incubated with 10 µL of 0.1 M glycine buffer (pH 2.5) and 0.05% BSA (w/v) for 2 min. Afterward, 20 µL of 0.25 M phosphate buffer (pH 7.6), 1 mM EDTA, and 0.05% BSA containing different concentrations of inhibitors was added to the samples. After incubation for 2 h at room temperature, 10 µL of sample was transferred into a Corning black polystyrene 96- well microplate (#CLS3925) containing 90 µL of 0.25 M phosphate buffer (pH 7.6), 1 mM EDTA, and 0.05% BSA (w/v) per well. The enzymatic reaction was initiated by addition of 20 µL of substrate buffer containing 18 mM pyruvate and 1.8 mM NADH for a final concentration of 3 mM pyruvate and 0.3 mM NADH. The consumption of NADH was followed over time by measuring the fluorescence emission intensity (Aexcitation = 340 nm, Aemission = 450 nm) with a SpectraMax m2e spectrophotometer (Molecular Devices). To calculate the inhibitory activity of the compounds, the fluorescence emission intensity of the samples was compared directly to a positive control without inhibitors before reaching the plateau phase of the reaction (70% of substrate consumed).

### 4.3. MST evaluation

MST measurements were performed according to a previously reported protocol on a Nanotemper Monolith NT.115 instrument (Nano-Temper Technologies) using Red-dyeNHS fluorescent labelling.⁴ LDH-5 and LDH-Htr purified to homogeneity were labelled with Monolith Red-dye-NHS second-generation labelling dye (Nanotemper Technologies), according to the supplied protocol. LDH-1, purified to homogeneity, was labelled using first generation labelling dye. Measurements were performed in 50 mM sodium phosphate, pH 7.6, and 100 mM NaCl containing 0.01% Tween-20 in standard-treated capillaries (Nano- Temper Technologies). The ligands (NADH and hits) were titrated in 1:1 dilutions following manufacturer's recommendations. Experiments were performed in triplicates using 40% LED power, medium MST power, laser on-time 20 s, and laser off time 3 s. Hits were evaluated for their thermophoretic pattern, and Kd's were extracted from raw data at a 1.5 se20 s MST on time depending on the hit MST profile.

### 4.4. Cellular evaluation

Cellular proliferation was evaluated as described previously.¹ MCF7 human breast cancer cells, MDA-MB-231 human breast adenocarcinoma or BJ normal human skin fibroblasts were routinely cultured in DMEM with 4.5 g/l glucose, 4 mM glutamine and 10 % FCS (full DMEM) at 37 °C in a humidified 5 % CO2 incubator. Assay media were either full DMEM or lactate enriched DMEM (1mM). Cells were seeded in a 96 wells plate at 2500 cells/well for MCF7 and MDA-MB-231 and 500 cells/well for BJ fibroblat. After 24hrs, compounds were added at 10X or 1X the enzymatic IC50 (1% DMSO, final concentration) and the cellular proliferation was monitored using a SpectraMax i3x Multi-Mode Microplate Reader (Molecular Devices) at 1, 3, 6, 24, 48 and 72hrs. Cells viability was also assessed after 72hrs using PrestoBlue^{™} Cell Viability Reagent following the manufacturer instruction.

### References

1. Brisson, L.; Banski, P.; Sboarina, M.; Dethier, C.; Danhier, P.; Fontenille, M. J.; Van Hee, V. F.; Vazeille, T.; Tardy, M.; Falces, J.; Bouzin, C.; Porporato, P. E.; Frederick, R.; Michiels, C.; Copetti, T.; Sonveaux, P., Lactate Dehydrogenase B Controls Lysosome Activity and Autophagy in Cancer. Cancer Cell 2016, 30 (3), 418-431.
2. Thabault, L.; Brisson, L.; Brustenga, C.; Gache, S. A. M.; Prevost, J. R. C.; Kozlova, A.; Spillier, Q.; Liberelle, M.; Benyahia, Z.; Messens, J.; Copetti, T.; Sonveaux, P.; Frederick, R., Interrogating the Lactate Dehydrogenase Tetramerization Site Using (Stapled) Peptides. Journal of Medicinal Chemistry 2020, 63 (9), 4628-4643.
3. Thabault, L.; Liberelle, M.; Koruza, K.; Yildiz, E.; Joudiou, N.; Messens, J.; Brisson, L.; Wouters, J.; Sonveaux, P.; Frederick, R., Discovery of a novel lactate dehydrogenase tetramerization domain using epitope mapping and peptides. J Biol Chem 2021, 296, 100422.
4. Thabault, L.; Brustenga, C.; Savoyen, P.; Van Gysel, M.; Wouters, J.; Sonveaux, P.; Frederick, R.; Liberelle, M., Discovery of small molecules interacting at lactate dehydrogenases tetrameric interface using a biophysical screening cascade. Eur J Med Chem 2022, 230, 114102.
5. Rani, R.; Kumar, V., Recent Update on Human Lactate Dehydrogenase Enzyme 5 (hLDH5) Inhibitors: A Promising Approach for Cancer Chemotherapy. Journal of Medicinal Chemistry 2016, 59 (2), 487-496.
6. Poli, G.; Granchi, C.; Aissaoui, M.; Minutolo, F.; Tuccinardi, T., Three-Dimensional Analysis of the Interactions between hLDH5 and Its Inhibitors. Molecules 2017, 22 (12), 2217.
7. Jafary, F.; Ganjalikhany, M. R.; Moradi, A.; Hemati, M.; Jafari, S., Novel Peptide Inhibitors for Lactate Dehydrogenase A (LDHA): A Survey to Inhibit LDHA Activity via Disruption of Protein-Protein Interaction. Sci Rep 2019, 9 (1), 4686.
8. Nadal-Bufi, F.; Mason, J. M.; Chan, L. Y.; Craik, D. J.; Kaas, Q.; Troeira Henriques, S., Designed beta-Hairpins Inhibit LDH5 Oligomerization and Enzymatic Activity. J Med Chem 2021, 64 (7), 3767-3779.
9. Nadal-Bufi, F.; Chan, L. Y.; Mohammad, H. H.; Mason, J. M.; Salomon, C.; Lai, A.; Thompson, E. W.; Craik, D. J.; Kaas, Q.; Henriques, S. T., Peptide-based LDH5 inhibitors enter cancer cells and impair proliferation. Cell Mol Life Sci 2022, 79 (12), 606.
10. Friberg, A.; Rehwinkel, H.; Nguyen, D.; Putter, V.; Quanz, M.; Weiske, J.; Eberspacher, U.; Heisler, I.; Langer, G., Structural Evidence for Isoform-Selective Allosteric Inhibition of Lactate Dehydrogenase A. ACS Omega 2020, 5 (22), 13034-13041.
11. Shibata, S.; Sogabe, S.; Miwa, M.; Fujimoto, T.; Takakura, N.; Naotsuka, A.; Kitamura, S.; Kawamoto, T.; Soga, T., identification of the first highly selective inhibitor of human lactate dehydrogenase B. Sci Rep 2021, 11 (1), 21353.
12. Woodford, M. R.; Baker-Williams, A. J.; Sager, R. A.; Backe, S. J.; Blanden, A. R.; Hashmi, F.; Kancherla, P.; Gori, A.; Loiselle, D. R.; Castelli, M.; Serapian, S. A.; Colombo, G.; Haystead, T. A.; Jensen, S. M.; Stetler-Stevenson, W. G.; Loh, S. N.; Schmidt, L. S.; Linehan, W. M.; Bah, A.; Bourboulia, D.; Bratslavsky, G.; Mollapour, M., The tumor suppressor folliculin inhibits lactate dehydrogenase A and regulates the Warburg effect. Nat Struct Mol Biol 2021, 28 (8), 662-670.
13. Dzierlenga, M. W.; Schwartz, S. D., Targeting a Rate-Promoting Vibration with an Allosteric Mediator in Lactate Dehydrogenase. J Phys Chem Lett 2016, 7 (13), 2591-6.
14. Andrews, B. A.; Dyer, R. B., Small molecule cores demonstrate non-competitive inhibition of lactate dehydrogenase. Medchemcomm 2018, 9 (8), 1369-1376.

## Claims

1. A compound of formula (1), (2) or (3), wherein
formula (1) is 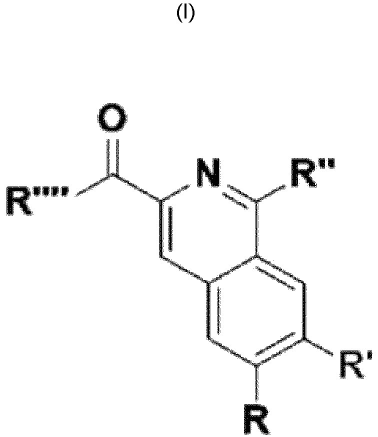 (formula (1)), wherein
R is -H, -F or -Cl;
R' is -H, -F or -Cl;
R" is 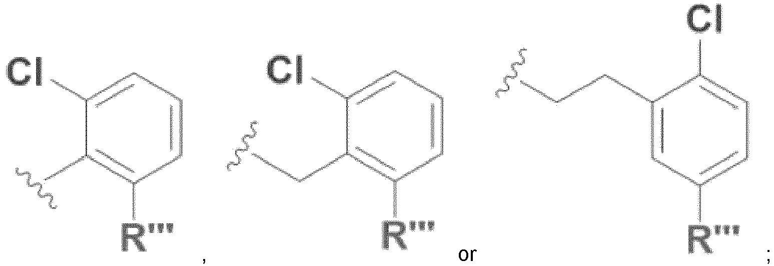
R‴ is -H or -OH; and
R"" is 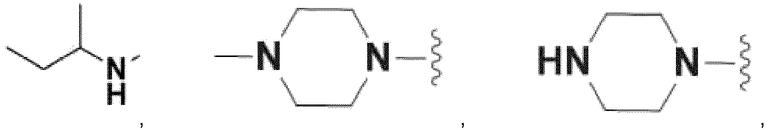 **n=** 1,2,3 n= 1,2,3 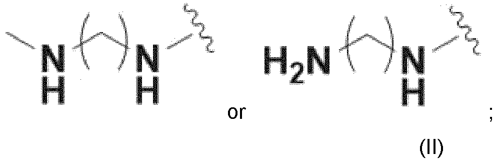 formula (2) is 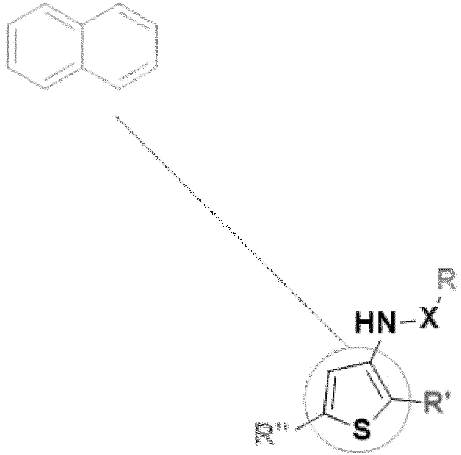
R is (formula (2)), wherein; 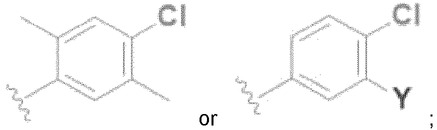
X is 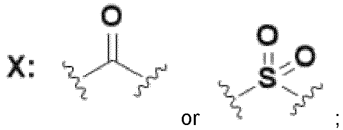
Y is -OH, -OCH₃ or CH₃;
R' is 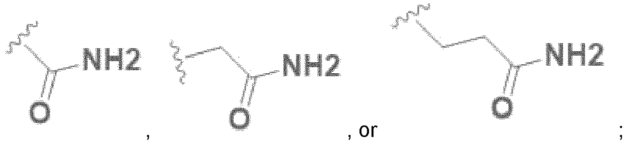 and
R" is 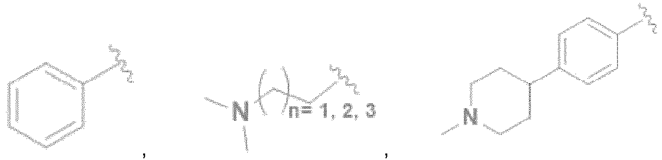
or 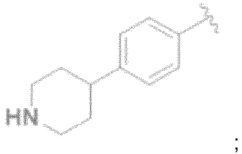 and
formula (III) is

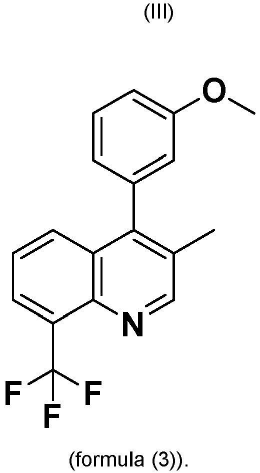

2. The compound of claim 1, wherein
to compound of formula (1) one or more of (i) to (v) apply:
(i) R is -H;
(ii) R' is -H;
(iii) R" is 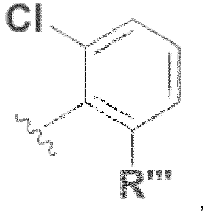 or;
(iv) R‴ is -H; and
(v) R"" is 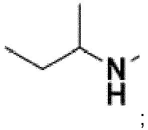
or
to compound of formula (2) one or more of (i) to (vi) apply:
(i) R is 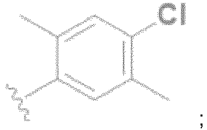
(ii) X is 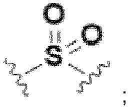
(iii) R' is 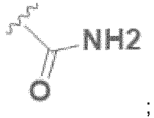 and
(iv) R" is

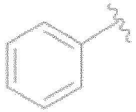

3. The compound of claim 1 or 2, wherein
the compound of formula (1) is 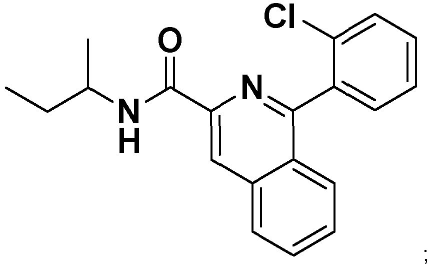 or
the compound of formula (2) is

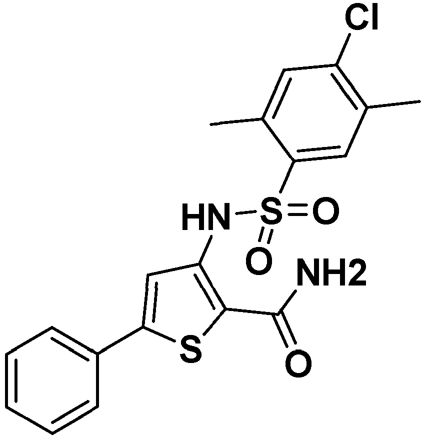

4. The compound of claim 1, wherein
(I)
the compound of formula (1) or (2) has a chemical structure being selected from the below list of compounds (1) to (191), preferably compounds (1) to (100), more preferably compounds (1) to (50), even more preferably compounds (1) to (25) and most preferably compounds (1) to (10)
(192) N-(aminomethyl)-7-chloro-1-(2-chloro-6-hydroxyphenyl)-6-fluoroisoquinoline-3-carboxamide
(193) N-(aminomethyl)-6,7-dichloro-1-(2-chloro-6-hydroxyphenyl)isoquinoline-3-carboxamide
(194) 7-chloro-1-(2-chloro-6-hydroxyphenyl)-6-fluoro-N-[2-(methylamino)ethyl]isoquinoline-3-carboxamide
(195) 7-chloro-1-(2-chloro-6-hydroxyphenyl)-6-fluoro-N-[3-(methylamino)propyl]isoquinoline-3-carboxamide
(196) N-(2-aminoethyl)-7-chloro-1-(2-chloro-6-hydroxyphenyl)-6-fluoroisoquinoline-3-carboxamide
(197) N-(3-aminopropyl)-7-chloro-1-(2-chloro-6-hydroxyphenyl)-6-fluoroisoquinoline-3-carboxamide
(198) 7-chloro-1-(2-chloro-6-hydroxyphenyl)-6-fluoro-N-[(methylamino)methyl]isoquinoline-3-carboxamide
(199) 6,7-dichloro-1-(2-chloro-6-hydroxyphenyl)-N-[3-(methylamino)propyl]isoquinoline-3-carboxamide
(200) 7-chloro-1-[(2-chloro-6-hydroxyphenyl)methyl]-6-fluoro-N-[3-(methylamino)propyl]isoquinoline-3-carboxamide
(201) 3-[3-(4-chloro-2,5-dimethylbenzenesulfonamido)-5-[4-(1-methylpiperidin-4-yl)phenyl]thiophen-2-yl]propanamide
(202) 3-chloro-2-[7-chloro-6-fluoro-3-(piperazine-1-carbonyl)isoquinolin-1-yl]phenol
(203) 6,7-dichloro-1-(2-chloro-6-hydroxyphenyl)-N-[2-(methylamino)ethyl]isoquinoline-3-carboxamide
(204) 3-chloro-2-[7-chloro-6-fluoro-3-(4-methylpiperazine-1-carbonyl)isoquinolin-1-yl]phenol
(205) N-(3-aminopropyl)-6,7-dichloro-1-(2-chloro-6-hydroxyphenyl)isoquinoline-3-carboxamide
(206) 3-chloro-2-[6,7-dichloro-3-(4-methylpiperazine-1-carbonyl)isoquinolin-1-yl]phenol
(207) N-(aminomethyl)-6,7-dichloro-1-[(2-chloro-6-hydroxyphenyl)methyl]isoquinoline-3-carboxamide
(208) N-(2-aminoethyl)-1-(2-chloro-6-hydroxyphenyl)-6,7-difluoroisoquinoline-3-carboxamide
(209) N-(2-aminoethyl)-6-chloro-1-(2-chloro-6-hydroxyphenyl)-7-fluoroisoquinoline-3-carboxamide
(210) N-[2-(carbamoylmethyl)-5-[4-(piperidin-4-yl)phenyl]thiophen-3-yl]-4-chloro-2,5-dimethylbenzamide
(211) 6-chloro-1-(2-chloro-6-hydroxyphenyl)-7-fluoro-N-[2-(methylamino)ethyl]isoquinoline-3-carboxamide
(212) 3-chloro-2-[6,7-dichloro-3-(piperazine-1-carbonyl)isoquinolin-1-yl]phenol
(213) N-(2-aminoethyl)-6,7-dichloro-1-(2-chloro-6-hydroxyphenyl)isoquinoline-3-carboxamide
(214) 6-chloro-1-(2-chloro-6-hydroxyphenyl)-7-fluoro-N-[(methylamino)methyl]isoquinoline-3-carboxamide
(215) 6,7-dichloro-1-(2-chloro-6-hydroxyphenyl)-N-[(methylamino)methyl]isoquinoline-3-carboxamide
(216) 3-chloro-2-[6-chloro-7-fluoro-3-(piperazine-1-carbonyl)isoquinolin-1-yl]phenol
(217) N-[2-(carbamoylmethyl)-5-[4-(1-methylpiperidin-4-yl)phenyl]thiophen-3-yl]-4-chloro-5-methoxy-2-methylbenzamide
(218) 4-chloro-3-{2-[6,7-difluoro-3-(piperazine-1-carbonyl)isoquinolin-1-yl]ethyl}phenol
(219) N-(3-aminopropyl)-6-chloro-1-(2-chloro-6-hydroxyphenyl)-7-fluoroisoquinoline-3-carboxamide
(220) N-[2-(carbamoylmethyl)-5-[4-(piperidin-4-yl)phenyl]thiophen-3-yl]-4-chloro-5-hydroxy-2-methylbenzamide
(221) N-(aminomethyl)-6-chloro-1-(2-chloro-6-hydroxyphenyl)-7-fluoroisoquinoline-3-carboxamide
(222) N-(3-aminopropyl)-1-(2-chloro-6-hydroxyphenyl)-6,7-difluoroisoquinoline-3-carboxamide
(223) 3-chloro-2-[6-chloro-7-fluoro-3-(4-methylpiperazine-1-carbonyl)isoquinolin-1-yl]phenol
(224) 6-chloro-1-(2-chloro-6-hydroxyphenyl)-7-fluoro-N-[3-(methylamino)propyl]isoquinoline-3-carboxamide
(225) 3-chloro-2-[6,7-difluoro-3-(piperazine-1-carbonyl)isoquinolin-1-yl]phenol
(226) 3-[3-(4-chloro-5-methoxy-2-methylbenzenesulfonamido)-5-[4-(piperidin-4-yl)phenyl]thiophen-2-yl]propanamide
(227) 3-chloro-2-{[6-chloro-7-fluoro-3-(piperazine-1-carbonyl)isoquinolin-1-yl]methyl}phenol
(228) N-[2-(carbamoylmethyl)-5-[4-(piperidin-4-yl)phenyl]thiophen-3-yl]-4-chloro-5-methoxy-2-methylbenzamide
(229) N-[2-(2-carbamoylethyl)-5-[4-(1-methylpiperidin-4-yl)phenyl]thiophen-3-yl]-4-chloro-5-hydroxy-2-methylbenzamide
(230) 6-chloro-1-[(2-chloro-6-hydroxyphenyl)methyl]-7-fluoro-N-[(methylamino)methyl]isoquinoline-3-carboxamide
(231) N-(aminomethyl)-1-[(2-chloro-6-hydroxyphenyl)methyl]-6,7-difluoroisoquinoline-3-carboxamide
(232) 6-chloro-1-[(2-chloro-6-hydroxyphenyl)methyl]-7-fluoro-N-[2-(methylamino)ethyl]isoquinoline-3-carboxamide
(233) N-[2-(carbamoylmethyl)-5-[4-(1-methylpiperidin-4-yl)phenyl]thiophen-3-yl]-4-chloro-5-hydroxy-2-methylbenzamide
(234) N-(3-aminopropyl)-1-[(2-chloro-6-hydroxyphenyl)methyl]-6,7-difluoroisoquinoline-3-carboxamide
(235) 6,7-dichloro-1-[(2-chloro-6-hydroxyphenyl)methyl]-N-[(methylamino)methyl]isoquinoline-3-carboxamide
(236) N-(aminomethyl)-1-(2-chloro-6-hydroxyphenyl)-6,7-difluoroisoquinoline-3-carboxamide
(237) 6-chloro-1-[(2-chloro-6-hydroxyphenyl)methyl]-7-fluoro-N-[3-(methylamino)propyl]isoquinoline-3-carboxamide
(238) N-(aminomethyl)-6-chloro-1-[(2-chloro-6-hydroxyphenyl)methyl]-7-fluoroisoquinoline-3-carboxamide
(239) 1-(2-chloro-6-hydroxyphenyl)-6,7-difluoro-N-[3-(methylamino)propyl]isoquinoline-3-carboxamide
(240) 7-chloro-1-[(2-chloro-6-hydroxyphenyl)methyl]-6-fluoro-N-[(methylamino)methyl]isoquinoline-3-carboxamide
(241) N-(aminomethyl)-7-chloro-1-[(2-chloro-6-hydroxyphenyl)methyl]-6-fluoroisoquinoline-3-carboxamide
(242) 3-chloro-2-{[6-chloro-7-fluoro-3-(4-methylpiperazine-1-carbonyl)isoquinolin-1-yl]methyl}phenol
(243) 1-[(2-chloro-6-hydroxyphenyl)methyl]-6,7-difluoro-N-[(methylamino)methyl]isoquinoline-3-carboxamide
(244) 3-(4-chloro-2,5-dimethylbenzenesulfonamido)-5-[4-(piperidin-4-yl)phenyl]thiophene-2-carboxamide
(245) 3-chloro-2-{[6,7-dichloro-3-(4-methylpiperazine-1-carbonyl)isoquinolin-1-yl]methyl}phenol
(246) N-(3-aminopropyl)-6,7-dichloro-1-[(2-chloro-6-hydroxyphenyl)methyl]isoquinoline-3-carboxamide
(247) 1-(2-chloro-6-hydroxyphenyl)-6,7-difluoro-N-[2-(methylamino)ethyl]isoquinoline-3-carboxamide
(248) 3-chloro-2-[6,7-difluoro-3-(4-methylpiperazine-1-carbonyl)isoquinolin-1-yl]phenol
(249) 1-(2-chloro-6-hydroxyphenyl)-6,7-difluoro-N-[(methylamino)methyl]isoquinoline-3-carboxamide
(250) N-(2-aminoethyl)-1-[(2-chloro-6-hydroxyphenyl)methyl]-6,7-difluoroisoquinoline-3-carboxamide
(251) 3-chloro-2-{[6,7-dichloro-3-(piperazine-1-carbonyl)isoquinolin-1-yl]methyl}phenol
(252) N-(2-aminoethyl)-7-chloro-1-[(2-chloro-6-hydroxyphenyl)methyl]-6-fluoroisoquinoline-3-carboxamide
(253) 2-[3-(4-chloro-5-methoxy-2-methylbenzenesulfonamido)-5-[4-(1-methylpiperidin-4-yl)phenyl]thiophen-2-yl]acetamide
(254) 3-chloro-2-{[7-chloro-6-fluoro-3-(piperazine-1-carbonyl)isoquinolin-1-yl]methyl}phenol
(255) 2-[3-(4-chloro-5-hydroxy-2-methylbenzenesulfonamido)-5-[4-(piperidin-4-yl)phenyl]thiophen-2-yl]acetamide
(256) 3-chloro-2-{[6,7-difluoro-3-(piperazine-1-carbonyl)isoquinolin-1-yl]methyl}phenol
(257) 3-chloro-2-{[7-chloro-6-fluoro-3-(4-methylpiperazine-1-carbonyl)isoquinolin-1-yl]methyl}phenol
(258) 7-chloro-1-[(2-chloro-6-hydroxyphenyl)methyl]-6-fluoro-N-[2-(methylamino)ethyl]isoquinoline-3-carboxamide
(259) 3-chloro-2-{[6,7-difluoro-3-(4-methylpiperazine-1-carbonyl)isoquinolin-1-yl]methyl}phenol
(260) 4-chloro-3-{2-[7-chloro-6-fluoro-3-(4-methylpiperazine-1-carbonyl)isoquinolin-1-yl]ethyl}phenol
(261) 6-chloro-1-[2-(2-chloro-5-hydroxyphenyl)ethyl]-7-fluoro-N-[3-(methylamino)propyl]isoquinoline-3-carboxamide
(262) 1-[(2-chloro-6-hydroxyphenyl)methyl]-6,7-difluoro-N-[2-(methylamino)ethyl]isoquinoline-3-carboxamide
(263) 3-[3-(4-chloro-5-hydroxy-2-methylbenzenesulfonamido)-5-[4-(piperidin-4-yl)phenyl]thiophen-2-yl]propanamide
(264) 1-[(2-chloro-6-hydroxyphenyl)methyl]-6,7-difluoro-N-[3-(methylamino)propyl]isoquinoline-3-carboxamide
(265) N-(aminomethyl)-7-chloro-1-[2-(2-chloro-5-hydroxyphenyl)ethyl]-6-fluoroisoquinoline-3-carboxamide
(266) N-[2-(carbamoylmethyl)-5-phenylthiophen-3-yl]-4-chloro-5-hydroxy-2-methylbenzamide
(267) N-[2-(2-carbamoylethyl)-5-[4-(piperidin-4-yl)phenyl]thiophen-3-yl]-4-chloro-2,5-dimethylbenzamide
(268) 6,7-dichloro-1-[(2-chloro-6-hydroxyphenyl)methyl]-N-[3-(methylamino)propyl]isoquinoline-3-carboxamide
(269) N-[2-(carbamoylmethyl)-5-[4-(1-methylpiperidin-4-yl)phenyl]thiophen-3-yl]-4-chloro-2,5-dimethylbenzamide
(270) 4-chloro-3-{2-[6-chloro-7-fluoro-3-(4-methylpiperazine-1-carbonyl)isoquinolin-1-yl]ethyl}phenol
(271) 3-[3-(4-chloro-2,5-dimethylbenzenesulfonamido)-5-[4-(piperidin-4-yl)phenyl]thiophen-2-yl]propanamide
(272) 3-(4-chloro-5-methoxy-2-methylbenzenesulfonamido)-5-phenylthiophene-2-carboxamide
(273) 3-[3-(4-chloro-5-hydroxy-2-methylbenzenesulfonamido)-5-[4-(1-methylpiperidin-4-yl)phenyl]thiophen-2-yl]propanamide
(274) N-[2-(carbamoylmethyl)-5-phenylthiophen-3-yl]-4-chloro-2,5-dimethylbenzamide
(275) N-[2-(2-carbamoylethyl)-5-[4-(1-methylpiperidin-4-yl)phenyl]thiophen-3-yl]-4-chloro-5-methoxy-2-methylbenzamide
(276) 3-(4-chloro-2,5-dimethylbenzamido)-5-[4-(1-methylpiperidin-4-yl)phenyl]thiophene-2-carboxamide
(277) 6-chloro-1-[2-(2-chloro-5-hydroxyphenyl)ethyl]-7-fluoro-N-[(methylamino)methyl]isoquinoline-3-carboxamide
(278) 4-chloro-3-{2-[6,7-dichloro-3-(piperazine-1-carbonyl)isoquinolin-1-yl]ethyl}phenol
(279) N-[2-(2-carbamoylethyl)-5-[4-(piperidin-4-yl)phenyl]thiophen-3-yl]-4-chloro-5-hydroxy-2-methylbenzamide
(280) 6,7-dichloro-1-[2-(2-chloro-5-hydroxyphenyl)ethyl]-N-[2-(methylamino)ethyl]isoquinoline-3-carboxamide
(281) 7-chloro-1-[2-(2-chloro-5-hydroxyphenyl)ethyl]-6-fluoro-N-[2-(methylamino)ethyl]isoquinoline-3-carboxamide
(282) 2-[3-(4-chloro-5-methoxy-2-methylbenzenesulfonamido)-5-[4-(piperidin-4-yl)phenyl]thiophen-2-yl]acetamide
(283) N-(2-aminoethyl)-6,7-dichloro-1-[(2-chloro-6-hydroxyphenyl)methyl]isoquinoline-3-carboxamide
(284) N-[2-(carbamoylmethyl)-5-[4-(dimethylamino)butyl]thiophen-3-yl]-4-chloro-2,5-dimethylbenzamide
(285) N-(3-aminopropyl)-6-chloro-1-[(2-chloro-6-hydroxyphenyl)methyl]-7-fluoroisoquinoline-3-carboxamide
(286) N-(aminomethyl)-6-chloro-1-[2-(2-chloro-5-hydroxyphenyl)ethyl]-7-fluoroisoquinoline-3-carboxamide
(287) N-(2-aminoethyl)-7-chloro-1-[2-(2-chloro-5-hydroxyphenyl)ethyl]-6-fluoroisoquinoline-3-carboxamide
(288) 6,7-dichloro-1-[2-(2-chloro-5-hydroxyphenyl)ethyl]-N-[(methylamino)methyl]isoquinoline-3-carboxamide
(289) N-(2-aminoethyl)-1-[2-(2-chloro-5-hydroxyphenyl)ethyl]-6,7-difluoroisoquinoline-3-carboxamide
(290) 3-(4-chloro-5-methoxy-2-methylbenzamido)-5-[4-(1-methylpiperidin-4-yl)phenyl]thiophene-2-carboxamide
(291) 1-[2-(2-chloro-5-hydroxyphenyl)ethyl]-6,7-difluoro-N-[2-(methylamino)ethyl]isoquinoline-3-carboxamide
(292) N-(2-aminoethyl)-6-chloro-1-[(2-chloro-6-hydroxyphenyl)methyl]-7-fluoroisoquinoline-3-carboxamide
(293) N-[2-(2-carbamoylethyl)-5-phenylthiophen-3-yl]-4-chloro-5-hydroxy-2-methylbenzamide
(294) N-(aminomethyl)-1-[2-(2-chloro-5-hydroxyphenyl)ethyl]-6,7-difluoroisoquinoline-3-carboxamide
(295) 7-chloro-1-[2-(2-chloro-5-hydroxyphenyl)ethyl]-6-fluoro-N-[3-(methylamino)propyl]isoquinoline-3-carboxamide
(296) N-(3-aminopropyl)-7-chloro-1-[2-(2-chloro-5-hydroxyphenyl)ethyl]-6-fluoroisoquinoline-3-carboxamide
(297) N-(2-aminoethyl)-6-chloro-1-[2-(2-chloro-5-hydroxyphenyl)ethyl]-7-fluoroisoquinoline-3-carboxamide
(298) 1-[2-(2-chloro-5-hydroxyphenyl)ethyl]-6,7-difluoro-N-[3-(methylamino)propyl]isoquinoline-3-carboxamide
(299) 4-chloro-3-{2-[6,7-difluoro-3-(4-methylpiperazine-1-carbonyl)isoquinolin-1-yl]ethyl}phenol
(300) N-(3-aminopropyl)-6-chloro-1-[2-(2-chloro-5-hydroxyphenyl)ethyl]-7-fluoroisoquinoline-3-carboxamide
(301) 1-[2-(2-chloro-5-hydroxyphenyl)ethyl]-6,7-difluoro-N-[(methylamino)methyl]isoquinoline-3-carboxamide
(302) 4-chloro-3-{2-[6,7-dichloro-3-(4-methylpiperazine-1-carbonyl)isoquinolin-1-yl]ethyl}phenol
(303) 3-[3-(4-chloro-5-methoxy-2-methylbenzenesulfonamido)-5-[4-(1-methylpiperidin-4-yl)phenyl]thiophen-2-yl]propanamide
(304) 2-[3-(4-chloro-2,5-dimethylbenzenesulfonamido)-5-[4-(piperidin-4-yl)phenyl]thiophen-2-yl]acetamide
(305) 3-(4-chloro-2,5-dimethylbenzamido)-5-[4-(piperidin-4-yl)phenyl]thiophene-2-carboxamide
(306) 6-chloro-1-[2-(2-chloro-5-hydroxyphenyl)ethyl]-7-fluoro-N-[2-(methylamino)ethyl]isoquinoline-3-carboxamide
(307) N-(3-aminopropyl)-6,7-dichloro-1-[2-(2-chloro-5-hydroxyphenyl)ethyl]isoquinoline-3-carboxamide
(308) N-[2-(2-carbamoylethyl)-5-[4-(1-methylpiperidin-4-yl)phenyl]thiophen-3-yl]-4-chloro-2,5-dimethylbenzamide
(309) 3-(4-chloro-5-methoxy-2-methylbenzamido)-5-[4-(piperidin-4-yl)phenyl]thiophene-2-carboxamide
(310) N-(2-aminoethyl)-6,7-dichloro-1-[2-(2-chloro-5-hydroxyphenyl)ethyl]isoquinoline-3-carboxamide
(311) 2-[3-(4-chloro-5-hydroxy-2-methylbenzenesulfonamido)-5-phenylthiophen-2-yl]acetamide
(312) 6,7-dichloro-1-[2-(2-chloro-5-hydroxyphenyl)ethyl]-N-[3-(methylamino)propyl]isoquinoline-3-carboxamide
(313) 3-(4-chloro-5-hydroxy-2-methylbenzamido)-5-phenylthiophene-2-carboxamide
(314) 3-(4-chloro-5-hydroxy-2-methylbenzenesulfonamido)-5-[4-(piperidin-4-yl)phenyl]thiophene-2-carboxamide
(315) 6,7-dichloro-1-[(2-chloro-6-hydroxyphenyl)methyl]-N-[2-(methylamino)ethyl]isoquinoline-3-carboxamide
(316) 3-(4-chloro-5-methoxy-2-methylbenzenesulfonamido)-5-[4-(1-methylpiperidin-4-yl)phenyl]thiophene-2-carboxamide
(317) 2-[3-(4-chloro-5-hydroxy-2-methylbenzenesulfonamido)-5-[4-(1-methylpiperidin-4-yl)phenyl]thiophen-2-yl]acetamide
(318) N-[2-(2-carbamoylethyl)-5-phenylthiophen-3-yl]-4-chloro-2,5-dimethylbenzamide
(319) N-[2-(2-carbamoylethyl)-5-[2-(dimethylamino)ethyl]thiophen-3-yl]-4-chloro-5-hydroxy-2-methylbenzamide
(320) 3-(4-chloro-5-methoxy-2-methylbenzamido)-5-phenylthiophene-2-carboxamide
(321) N-(3-aminopropyl)-1-[2-(2-chloro-5-hydroxyphenyl)ethyl]-6,7-difluoroisoquinoline-3-carboxamide
(322) 2-[3-(4-chloro-2,5-dimethylbenzenesulfonamido)-5-[4-(1-methylpiperidin-4-yl)phenyl]thiophen-2-yl]acetamide
(323) 3-(4-chloro-5-hydroxy-2-methylbenzamido)-5-[4-(piperidin-4-yl)phenyl]thiophene-2-carboxamide
(324) 7-chloro-1-[2-(2-chloro-5-hydroxyphenyl)ethyl]-6-fluoro-N-[(methylamino)methyl]isoquinoline-3-carboxamide
(325) 4-chloro-3-{2-[7-chloro-6-fluoro-3-(piperazine-1-carbonyl)isoquinolin-1-yl]ethyl}phenol
(326) N-[2-(2-carbamoylethyl)-5-[4-(piperidin-4-yl)phenyl]thiophen-3-yl]-4-chloro-5-methoxy-2-methylbenzamide
(327) 3-(4-chloro-2,5-dimethylbenzenesulfonamido)-5-[4-(1-methylpiperidin-4-yl)phenyl]thiophene-2-carboxamide
(328) N-[2-(2-carbamoylethyl)-5-[2-(dimethylamino)ethyl]thiophen-3-yl]-4-chloro-2,5-dimethylbenzamide
(329) N-[2-(2-carbamoylethyl)-5-[3-(dimethylamino)propyl]thiophen-3-yl]-4-chloro-2,5-dimethylbenzamide
(330) 3-(4-chloro-5-hydroxy-2-methylbenzamido)-5-[4-(1-methylpiperidin-4-yl)phenyl]thiophene-2-carboxamide
(331) 2-[3-(4-chloro-5-hydroxy-2-methylbenzenesulfonamido)-5-[2-(dimethylamino)ethyl]thiophen-2-yl]acetamide
(332) 2-[3-(4-chloro-5-hydroxy-2-methylbenzenesulfonamido)-5-[3-(dimethylamino)propyl]thiophen-2-yl]acetamide
(333) N-[2-(carbamoylmethyl)-5-[3-(dimethylamino)propyl]thiophen-3-yl]-4-chloro-2,5-dimethylbenzamide
(334) 3-(4-chloro-5-hydroxy-2-methylbenzenesulfonamido)-5-[4-(dimethylamino)butyl]thiophene-2-carboxamide
(335) 3-(4-chloro-2,5-dimethylbenzenesulfonamido)-5-phenylthiophene-2-carboxamide
(336) 3-[3-(4-chloro-2,5-dimethylbenzenesulfonamido)-5-phenylthiophen-2-yl]propanamide
(337) N-[2-(2-carbamoylethyl)-5-[3-(dimethylamino)propyl]thiophen-3-yl]-4-chloro-5-methoxy-2-methylbenzamide
(338) 3-(4-chloro-5-hydroxy-2-methylbenzamido)-5-[4-(dimethylamino)butyl]thiophene-2-carboxamide
(339) 2-[3-(4-chloro-5-hydroxy-2-methylbenzenesulfonamido)-5-[4-(dimethylamino)butyl]thiophen-2-yl]acetamide
(340) 3-(4-chloro-5-hydroxy-2-methylbenzamido)-5-[3-(dimethylamino)propyl]thiophene-2-carboxamide
(341) 3-(4-chloro-2,5-dimethylbenzamido)-5-[2-(dimethylamino)ethyl]thiophene-2-carboxamide
(342) N-(aminomethyl)-6,7-dichloro-1-[2-(2-chloro-5-hydroxyphenyl)ethyl]isoquinoline-3-carboxamide
(343) 3-(4-chloro-2,5-dimethylbenzamido)-5-[3-(dimethylamino)propyl]thiophene-2-carboxamide
(344) 3-(4-chloro-5-hydroxy-2-methylbenzenesulfonamido)-5-[2-(dimethylamino)ethyl]thiophene-2-carboxamide
(345) N-[2-(2-carbamoylethyl)-5-[4-(dimethylamino)butyl]thiophen-3-yl]-4-chloro-2,5-dimethylbenzamide
(346) 3-(4-chloro-5-hydroxy-2-methylbenzamido)-5-[2-(dimethylamino)ethyl]thiophene-2-carboxamide
(347) 3-[3-(4-chloro-2,5-dimethylbenzenesulfonamido)-5-[2-(dimethylamino)ethyl]thiophen-2-yl]propanamide
(348) 3-[3-(4-chloro-5-methoxy-2-methylbenzenesulfonamido)-5-[3-(dimethylamino)propyl]thiophen-2-yl]propanamide
(349) 3-(4-chloro-2,5-dimethylbenzenesulfonamido)-5-[3-(dimethylamino)propyl]thiophene-2-carboxamide
(350) N-[2-(carbamoylmethyl)-5-[3-(dimethylamino)propyl]thiophen-3-yl]-4-chloro-5-hydroxy-2-methylbenzamide
(351) 3-(4-chloro-5-hydroxy-2-methylbenzenesulfonamido)-5-phenylthiophene-2-carboxamide
(352) 3-[3-(4-chloro-5-methoxy-2-methylbenzenesulfonamido)-5-[4-(dimethylamino)butyl]thiophen-2-yl]propanamide
(353) N-[2-(carbamoylmethyl)-5-[2-(dimethylamino)ethyl]thiophen-3-yl]-4-chloro-2,5-dimethylbenzamide
(354) N-[2-(2-carbamoylethyl)-5-[2-(dimethylamino)ethyl]thiophen-3-yl]-4-chloro-5-methoxy-2-methylbenzamide
(355) 3-(4-chloro-2,5-dimethylbenzenesulfonamido)-5-[4-(dimethylamino)butyl]thiophene-2-carboxamide
(356) 2-[3-(4-chloro-2,5-dimethylbenzenesulfonamido)-5-[2-(dimethylamino)ethyl]thiophen-2-yl]acetamide
(357) 3-[3-(4-chloro-5-methoxy-2-methylbenzenesulfonamido)-5-[2-(dimethylamino)ethyl]thiophen-2-yl]propanamide
(358) N-(3-aminopropyl)-7-chloro-1-[(2-chloro-6-hydroxyphenyl)methyl]-6-fluoroisoquinoline-3-carboxamide
(359) N-[2-(carbamoylmethyl)-5-[2-(dimethylamino)ethyl]thiophen-3-yl]-4-chloro-5-methoxy-2-methylbenzamide
(360) N-[2-(2-carbamoylethyl)-5-phenylthiophen-3-yl]-4-chloro-5-methoxy-2-methylbenzamide
(361) 3-(4-chloro-2,5-dimethylbenzamido)-5-[4-(dimethylamino)butyl]thiophene-2-carboxamide
(362) N-[2-(carbamoylmethyl)-5-phenylthiophen-3-yl]-4-chloro-5-methoxy-2-methylbenzamide
(363) 3-[3-(4-chloro-5-hydroxy-2-methylbenzenesulfonamido)-5-[2-(dimethylamino)ethyl]thiophen-2-yl]propanamide
(364) N-[2-(2-carbamoylethyl)-5-[4-(dimethylamino)butyl]thiophen-3-yl]-4-chloro-5-hydroxy-2-methylbenzamide
(365) N-[2-(carbamoylmethyl)-5-[2-(dimethylamino)ethyl]thiophen-3-yl]-4-chloro-5-hydroxy-2-methylbenzamide
(366) 3-[3-(4-chloro-2,5-dimethylbenzenesulfonamido)-5-[4-(dimethylamino)butyl]thiophen-2-yl]propanamide
(367) 3-(4-chloro-2,5-dimethylbenzenesulfonamido)-5-[2-(dimethylamino)ethyl]thiophene-2-carboxamide
(368) 2-[3-(4-chloro-2,5-dimethylbenzenesulfonamido)-5-[4-(dimethylamino)butyl]thiophen-2-yl]acetamide
(369) 3-[3-(4-chloro-2,5-dimethylbenzenesulfonamido)-5-[3-(dimethylamino)propyl]thiophen-2-yl]propanamide
(370) N-[2-(carbamoylmethyl)-5-[4-(dimethylamino)butyl]thiophen-3-yl]-4-chloro-5-methoxy-2-methylbenzamide
(371) N-[2-(carbamoylmethyl)-5-[3-(dimethylamino)propyl]thiophen-3-yl]-4-chloro-5-methoxy-2-methylbenzamide
(372) 3-(4-chloro-2,5-dimethylbenzamido)-5-phenylthiophene-2-carboxamide
(373) 3-(4-chloro-5-methoxy-2-methylbenzamido)-5-[2-(dimethylamino)ethyl]thiophene-2-carboxamide
(374) 2-[3-(4-chloro-2,5-dimethylbenzenesulfonamido)-5-phenylthiophen-2-yl]acetamide
(375) N-[2-(2-carbamoylethyl)-5-[3-(dimethylamino)propyl]thiophen-3-yl]-4-chloro-5-hydroxy-2-methylbenzamide
(376) 3-[3-(4-chloro-5-hydroxy-2-methylbenzenesulfonamido)-5-phenylthiophen-2-yl]propanamide
(377) 3-(4-chloro-5-methoxy-2-methylbenzenesulfonamido)-5-[3-(dimethylamino)propyl]thiophene-2-carboxamide
(378) 2-[3-(4-chloro-2,5-dimethylbenzenesulfonamido)-5-[3-(dimethylamino)propyl]thiophen-2-yl]acetamide
(379) N-[2-(carbamoylmethyl)-5-[4-(dimethylamino)butyl]thiophen-3-yl]-4-chloro-5-hydroxy-2-methylbenzamide
(380) 3-[3-(4-chloro-5-methoxy-2-methylbenzenesulfonamido)-5-phenylthiophen-2-yl]propanamide
(381) N-[2-(2-carbamoylethyl)-5-[4-(dimethylamino)butyl]thiophen-3-yl]-4-chloro-5-methoxy-2-methylbenzamide
(382) 4-chloro-3-{2-[6-chloro-7-fluoro-3-(piperazine-1-carbonyl)isoquinolin-1-yl]ethyl}phenol

5. The compound of any one of claims 1 to 4, wherein the compound is an inhibitor of LDH (lactate dehydrogenase, E.C. 1.1.1.27).

6. The compound of claim 5, wherein the inhibitor inhibits the formation of the enzymatically active tetrameric form of LDH.

7. The compound of claim 5 or 6, wherein the LDH inhibitor displays an IC₅₀ for LDH of 100 µM of less, preferably 50µM or less for human LDHA and/or LDHB.

8. The compound of any one of claims 5 to 7, wherein the LDH
(i) comprises or consists of the amino acid sequence of SEQ ID NO: 1 or 2, or a sequence being at least 90% identical thereto; or
(ii) comprises or consists of an amino acid sequence being encoded by the nucleotide sequence of SEQ ID NO: 3 or 4, or a sequence being at least 90% identical thereto.

9. A conjugate comprising the compound of any one of claims 1 to 8 conjugated to a peptide or protein, preferably a pharmaceutically active peptide or protein.

10. The conjugate of claim 9, wherein the peptide or protein is an antibody or an antibody mimetic, preferably an anti-tumor antigen antibody or antibody mimetic.

11. A pharmaceutical composition comprising the compound of any one of claims 1 to 8 or the conjugate of claim 9 or 10.

12. The compound, conjugate or the pharmaceutical composition of any preceding claims for use as a medicament.

13. The compound, conjugate or the pharmaceutical composition of any preceding claims for use in treating a cell proliferative disease.

14. The compound, conjugate or the pharmaceutical composition for use of claim 13, wherein the cell proliferative disease is cancer.

15. The compound, conjugate or the pharmaceutical composition for use of claim 14, wherein the cancer is selected from breast cancer, ovarian cancer, endometrial cancer, vaginal cancer, vulva cancer, bladder cancer, salivary gland cancer, pancreatic cancer, thyroid cancer, kidney cancer, lung cancer, cancer concerning the upper gastrointestinal tract, colon cancer, colorectal cancer, prostate cancer, squamous-cell carcinoma of the head and neck, cervical cancer, glioblastomas, malignant ascites, lymphomas and leukemias.
